# EUROPEAN PATENT APPLICATION

(11) **EP 3 604 492 A1**
(43) Date of publication of application: **05.02.2020**
(21) Application number: 18775507.9
(22) Date of filing: 29.03.2018
(51) Int. Cl.: C12M 1/00, G01N 35/00, G01N 35/10

(54) **NUCLEIC ACID SEPARATION APPARATUS**

(30) Priority: 31.03.2017 JP 2017072392; 31.03.2017 JP 2017072404
(71) Applicant: Kurashiki Boseki Kabushiki Kaisha, Kurashiki-shi Okayama 710-0054 (JP)
(72) Inventor: WATANABE, Kengo, Neyagawa-shi Osaka 572-0823 (JP); NAKAGAWA, Yasushi, Neyagawa-shi Osaka 572-0823 (JP)
(74) Representative: Hoffmann Eitle
(86) International application number: PCT/JP2018/013252
(87) International publication number: WO 2018/181718

(57) **Abstract**

A nucleic acid separating apparatus includes: a stirring mechanism that stirs and resolving-processes a specimen and a reagent injected into a process tube; a pressurized gas supply mechanism that supplies a pressurized gas to a filter cartridge having a specimen liquid including nucleic acid, the specimen liquid being injected in the filter cartridge after the resolving-process, to separate and collect nucleic acid from the specimen liquid; a dispensing mechanism that injects the specimen, a reagent, or the specimen liquid after the resolving-process into the process tube and the filter cartridge; and a move mechanism that moves the pressurized gas supply mechanism and the dispensing mechanism, wherein the dispensing mechanism includes: a suction and discharge mechanism that is capable of attaching a dispensing chip to the suction and discharge mechanism and detaching the dispensing chip from the suction and discharge mechanism, the suction and discharge mechanism being capable of suctioning and discharging a liquid through the dispensing chip; and a liquid supply mechanism that discharges a liquid from a nozzle.

## Description

### TECHNICAL FIELD

The present invention relates to a nucleic acid separating apparatus that automatically executes steps from pre-process steps for a specimen liquid including nucleic acid to a nucleic acid extraction step.

### BACKGROUND ART

A technique has recently been widely used according to which nucleic acid (mainly DNA) storing therein the gene information of life is extracted from the blood of a human, a tissue of an animal or a plant, or the like and is analyzed. Various dedicated reagents, apparatuses, and the like are used to extract DNA from blood, a tissue, or the like.

Automation has recently been advanced for a nucleic acid extraction step (see, e.g., Patent Document 1: Japanese Patent Publication No. 3635645). On the other hand, many reagents need to be handled at the what-is-called pre-process steps at which reagents are added to blood, a tissue or the like, and these items are stirred, and the like. The pre-process steps include various types of work such as the addition of reagents, stirring, and pressurizing. Any automation of these steps has not yet been advanced and these steps are still manually performed.

### SUMMARY

For handling the blood, the tissue, and the like, it is necessary to avoid any infection caused by contamination, flying about, or the like, and it has therefore been desired to reduce the manual work to shift to automated work as much as possible.

At the pre-process steps, however, the plural reagents need to be handled. The pre-process steps include many steps for a resolving process such as the reagent addition step, the stirring step, and a warming step, and any automation of the pre-process steps has been considered to be difficult.

An object of the present invention is to provide an automatic nucleic acid separating apparatus capable of automating the pre-process steps such as the dissolving process that have traditionally been manually performed.

In one general aspect, the techniques discloses here feature: a nucleic acid separating apparatus includes:
a stirring mechanism that stirs and resolving-processes a specimen and a reagent injected into a process tube;
a pressurized gas supply mechanism that supplies a pressurized gas to a filter cartridge having a specimen liquid including nucleic acid, the specimen liquid being injected in the filter cartridge after the resolving-process, to separate and collect nucleic acid from the specimen liquid;
a dispensing mechanism that injects the specimen, a reagent, or the specimen liquid after the resolving-process into the process tube and the filter cartridge; and
a move mechanism that moves the pressurized gas supply mechanism and the dispensing mechanism,
wherein the dispensing mechanism includes:
   a suction and discharge mechanism that is capable of attaching a dispensing chip to the suction and discharge mechanism and detaching the dispensing chip from the suction and discharge mechanism, the suction and discharge mechanism being capable of suctioning and discharging a liquid through the dispensing chip; and
   a liquid supply mechanism that discharges a liquid from a nozzle.

According to the nucleic acid separating apparatus of the present invention, plural steps such as dispensing and pressurizing can be automated, and not only the nucleic acid extraction step but also the traditionally manually performed pre-process steps can be automated.

### BRIEF DESCRIPTION OF DRAWINGS

Fig. 1A is a perspective schematic plan diagram of the planar configuration of a nucleic acid separating apparatus according to a first embodiment seen through a top board thereof.
Fig. 1B is a perspective schematic plan diagram of the configuration of the nucleic acid separating apparatus in Fig. 1A seen through a front panel thereof.
Fig. 2 is a block diagram of the configuration of the nucleic acid separating apparatus according to the first embodiment.
Fig. 3A is a plan diagram of the overall configuration of a dispensing chip/reagent setting part of the nucleic acid separating apparatus in Fig. 1.
Fig. 3B is a plan diagram for explaining the configuration in a reagent container tray of the dispensing chip/reagent setting part of the nucleic acid separating apparatus in Fig. 1.
Fig. 4A is a plan diagram of the configuration of a sample setting part of the nucleic acid separating apparatus in Fig. 1.
Fig. 4B is a diagram of an example of sample containers arranged in the sample setting part in Fig. 4A.
Fig. 5A is a plan diagram of the configuration of a warming/stirring part of the nucleic acid separating apparatus in Fig. 1.
Fig. 5B is a front diagram of Fig. 5A.
Fig. 5C is a perspective diagram of the structure of each of process tubes to be arranged in the warming/stirring part in Fig. 5A.
Fig. 6 is a plan diagram of the state where the process tubes are stirred in the warming/stirring part.
Fig. 7 is a front diagram of the warming/stirring part that warms the process tubes.
Fig. 8A is a plan diagram of the configuration of a nucleic acid extracting part of the nucleic acid separating apparatus in Fig. 1.
Fig. 8B is a front diagram of Fig. 8A.
Fig. 8C is a schematic perspective diagram of the configuration of a filter cartridge to be arranged in the nucleic acid extracting part in Fig. 8A.
Fig. 8D is a schematic perspective diagram of the configuration of a collection tube to be arranged in the nucleic acid extracting part 14 in Fig. 8A.
Fig. 9A is a plan diagram of the state where the filter cartridge is moved onto the collection tube for collecting nucleic acid to separate nucleic acid, in the nucleic acid extracting part.
Fig. 9B is a front diagram of Fig. 9A.
Fig. 10A is a schematic perspective diagram of the configuration of a dispensing and pressurizing unit of the nucleic acid separating apparatus in Fig. 1.
Fig. 10B is a partial diagram of liquid supply nozzles of the dispensing and pressurizing unit in Fig. 10A.
Fig. 10C is a partial diagram of pressurizing heads of the dispensing and pressurizing unit in Fig. 10A.
Fig. 10D is a partial diagram of suction/discharge nozzles of the dispensing and pressurizing unit in Fig. 10A.
Fig. 11A is a plan diagram of the dispensing and pressurizing unit.
Fig. 11B is a side diagram of the dispensing and pressurizing unit.
Fig. 11C is a front diagram of the dispensing and pressurizing unit.
Fig. 12A is a plan diagram of a liquid supply nozzles in the dispensing and pressurizing unit, and tube pumps and reagent bottles connected to the liquid supply nozzles.
Fig. 12B is a side diagram of the dispensing and pressurizing unit.
Fig. 12C is a front diagram of the dispensing and pressurizing unit.
Fig. 13A is a plan diagram of the pressurizing heads in the dispensing and pressurizing unit, and pipes connected to the pressurizing heads.
Fig. 13B is a side diagram of the dispensing and pressurizing unit.
Fig. 13C is a front diagram of the dispensing and pressurizing unit.
Fig. 14 is a flowchart of steps of a nucleic acid separation method according to the first embodiment.
Fig. 15A is a schematic perspective diagram of reagent suction of a reagent addition step S01 in Fig. 14.
Fig. 15B is a schematic perspective diagram of dispensing of the reagent into the process tube.
Fig. 16A is a schematic perspective diagram of blood suction at a blood addition step S02 in Fig. 14.
Fig. 16B is a schematic perspective diagram of dispensing the blood into the process tube.
Fig. 17A is a schematic perspective diagram of reagent suction at a reagent addition step S03 in Fig. 14.
Fig. 17B is a schematic perspective diagram of dispensing of the reagent into the process tube.
Fig. 18 is a schematic perspective diagram of the state where the process tube is stirred at a stirring step S04 in Fig. 14.
Fig. 19 is a schematic perspective diagram of the state where the process tube is warmed at a warming step S05 in Fig. 14.
Fig. 20A is a schematic perspective diagram of reagent suction at a reagent addition step S06 in Fig. 14.
Fig. 20B is a schematic perspective diagram of dispensing of the reagent into the process tube.
Fig. 21 is a schematic perspective diagram of the state where the process tube is stirred at a stirring step S07 in Fig. 14.
Fig. 22A is a schematic perspective diagram of suction from the process tubes at a liquid move step S08 in Fig. 14.
Fig. 22B is a schematic perspective diagram of dispensing into the filter cartridges.
Fig. 23 is a schematic perspective diagram of the state where the inside of each of the filter cartridges is pressurized at pressurizing step S09 in Fig. 14.
Fig. 24 is a schematic perspective diagram of the state where a cleaning reagent is dispensed into the filter cartridges at a reagent addition step S10 in Fig. 14.
Fig. 25 is a schematic perspective diagram of the state where the inside of each of the filter cartridge is pressurized at a pressurizing step S11 in Fig. 14.
Fig. 26 is a schematic perspective diagram of the state where the cleaning reagent is dispensed into the filter cartridges at a reagent addition step S12 in Fig. 14.
Fig. 27 is a schematic perspective diagram of the state where the inside of each of the filter cartridges is pressurized at a pressurizing step S13 in Fig. 14.
Fig. 28 is a schematic perspective diagram of the state where the cleaning reagent is dispensed into each of the filter cartridges at a reagent addition step S14 in Fig. 14.
Fig. 29 is a schematic perspective diagram of the state where the inside of each of the filter cartridges is pressurized at a pressurizing step S15 in Fig. 14.
Fig. 30A is a schematic perspective diagram of reagent (collection liquid) suction of a reagent addition step S16 in Fig. 14.
Fig. 30B is a schematic perspective diagram of dispensing of the reagent (the collection liquid) into the filter cartridges.
Fig. 31 is a schematic perspective diagram of the state where the inside of each of the filter cartridges is pressurized at a pressurizing step S17 in Fig. 14.
Fig. 32 is a block diagram of the configuration of a nucleic acid separating apparatus according to a second embodiment.
Fig. 33 is a side diagram of a sample rack presenting a bar code on a side face of each sample container and a bar code indicating each position of the sample rack.
Fig. 34A is a plan diagram of the state where a sample ID is read when one sample rack is stored in a sample setting part in Fig. 4A.
Fig. 34B is a front diagram of Fig. 34A.
Fig. 34C is a schematic diagram of the state where the bar code on the side face of the sample container is read by a bar code reader.
Fig. 35A is plan diagram of an ID reading dedicated lane for reading the collection tube ID of the collection tube in the nucleic acid extracting part in Fig. 8A.
Fig. 35B is a front diagram of an installation point for a code reader in the ID reading dedicated lane in Fig. 35A.
Fig. 35C is a bottom diagram of the collection tube rack installed in the ID reading dedicated lane in Fig. 35A.
Fig. 35D is a schematic diagram of the state where a two-dimensional code of the collection tube is read by a code reader.
Fig. 36A is a plan diagram of a modification example that reads the collection tube ID without disposing the ID reading dedicated lane.
Fig. 36B is a front diagram of an installation point for the code reader in the modification example in Fig. 36A.
Fig. 37 is a flowchart of steps of a nucleic acid separation method according to the second embodiment.
Fig. 38 is a schematic diagram of the state where the bar code on a side face of the sample container is read by the code reader.
Fig. 39 is a schematic diagram of the state where a two-dimensional code of the collection tube is read by the code reader.
Fig. 40 is a schematic perspective diagram of blood suction at a blood addition step in Fig. 37.

### DETAILED DESCRIPTION

A nucleic acid separating apparatus according to a first aspect includes:
a stirring mechanism that stirs and resolving-processes a specimen and a reagent injected into a process tube;
a pressurized gas supply mechanism that supplies a pressurized gas to a filter cartridge having a specimen liquid including nucleic acid, the specimen liquid being injected in the filter cartridge after the resolving-process, to separate and collect nucleic acid from the specimen liquid
a dispensing mechanism that injects the specimen, a reagent, or the specimen liquid after the resolving-process into the process tube and the filter cartridge; and
a move mechanism that moves the pressurized gas supply mechanism and the dispensing mechanism,
wherein the dispensing mechanism includes:
   a suction and discharge mechanism that is capable of attaching a dispensing chip to the suction and discharge mechanism and detaching the dispensing chip from the suction and discharge mechanism, the suction and discharge mechanism being capable of suctioning and discharging a liquid through the dispensing chip; and
   a liquid supply mechanism that discharges a liquid from a nozzle.

Further, as a nucleic acid separating apparatus of a second aspect, in the first aspect, the suction and discharge mechanism suctions or discharges a gas in the dispensing chip, and suctions or discharges a liquid into the dispensing chip, and
wherein the suction and discharge mechanism includes:
a first dispensing chip attaching part that includes an O-ring having a first caliber at a tip of the first dispensing chip attaching part, the first dispensing chip attaching part being capable of attaching a first dispensing chip having the first caliber to the first dispensing chip attaching part and detaching the first dispensing chip from the first dispensing chip attaching part; and;
a second dispensing chip attaching part that includes an O-ring having a second caliber larger than the first caliber to be concentric with the O-ring having the first caliber, the second dispensing chip attaching part being capable of attaching a second dispensing chip having the second caliber to the second dispensing chip attaching part and detaching the second dispensing chip from the second dispensing chip attaching part..

A nucleic acid separating apparatus according to a third aspect includes:
a stirring mechanism that stirs and resolving-processes a specimen and a reagent injected into a process tube;
a pressurized gas supply mechanism that supplies a pressurized gas to a filter cartridge having a specimen liquid including nucleic acid, the specimen liquid being injected in the filter cartridge after the resolving-process, to separate and collect nucleic acid from the specimen liquid to a collection tube;
a dispensing mechanism that injects the specimen, a reagent, or the specimen liquid after the resolving-process into the process tube and the filter cartridge; and
a move mechanism that moves the pressurized gas supply mechanism and the dispensing mechanism,
a sample ID reading part that reads a sample ID of a sample container including the specimen;
a collection tube ID reading part that reads a collection tube ID of the collection tube; and
an ID managing part that manages the sample ID and the corresponding collection tube ID correlating these with each other.

Further, as a nucleic acid separating apparatus of a fourth aspect, in the third aspect, further may include a sample rack that stores the plural sample containers in row,
wherein a sample position ID may be provided at the position for each point to store the sample container in the each point of the sample rack, the sample position ID indicating the position for storing the sample container,
wherein a sample ID reading part may read the sample ID and the sample position ID from the direction that intersects the setting direction of the sample rack,
wherein the ID managing part may correlate the sample ID and the corresponding sample position ID each other.

Further, as a nucleic acid separating apparatus of a fifth aspect, in the third or fourth aspect, further may include a collection tube rack that stores the plural collection tubes in row,
wherein a collection tube position ID may be provided at the position for each point to store the collection tube in the each point of the collection tube rack, the collection tube position ID indicating the position for storing the collection tube,
wherein a collection tube ID reading part may read the collection tube ID and the collection tube position ID from the direction that intersects the setting direction of the collection tube rack,
wherein the ID managing part may correlate the collection tube ID and the corresponding collection tube position ID each other.

A nucleic acid separating apparatus according to each of embodiments will be described below with reference to the accompanying drawings. In the drawings, substantially same members are given the same reference numerals.

### (First Embodiment)

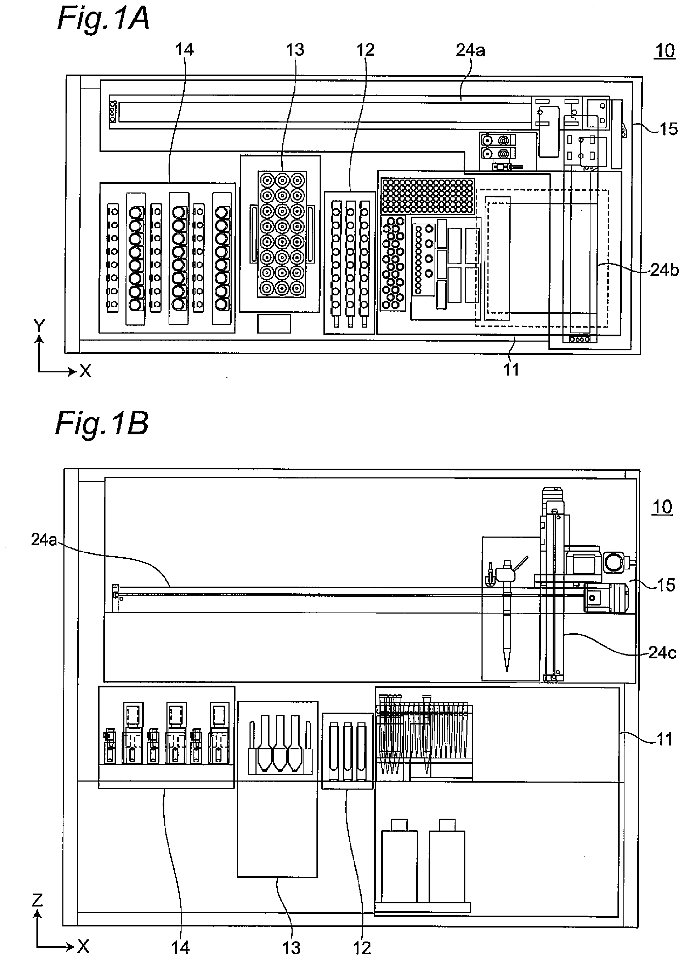

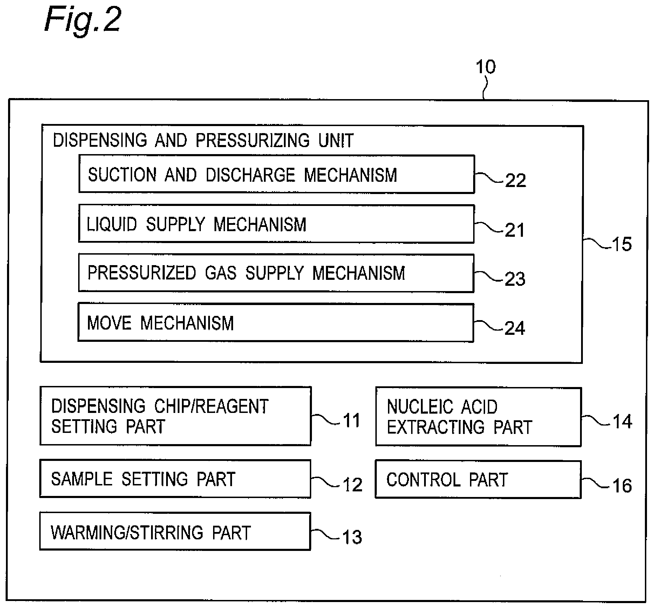

Fig. 1A is a perspective schematic plan diagram of the planar configuration of the nucleic acid separating apparatus 10 according to the first embodiment seen through a top board thereof. Fig. 1B is a perspective schematic plan diagram of the configuration of the nucleic acid separating apparatus 10 in Fig. 1A seen through a front panel thereof. Fig. 2 is a block diagram of the configuration of the nucleic acid separating apparatus 10 according to the first embodiment. The nucleic acid separating apparatus 10 includes a dispensing chip/reagent setting part 11, a sample setting part 12, a warming/stirring part 13, a nucleic acid extracting part 14, a dispensing and pressurizing unit 15, and a control part 16.

As depicted in Fig. 1A, Fig. 1B, and Fig. 2, the nucleic acid separating apparatus 10 includes the warming/stirring part 13 including a stirring mechanism 51 that stirs and resolving-processes a process tube 53 that has a specimen and a reagent injected therein, a pressured gas supply mechanism 23 supplying a pressurized gas to a filter cartridge 63 that has a specimen liquid injected therein to separate and collect nucleic acid from the specimen liquid including nucleic acid after a resolving process, a dispensing mechanism 21 and 22 injecting the specimen or the specimen liquid after the resolving process into the process tube 53 and a filter cartridge 63, and a move mechanism 24 moving the pressurized gas supply mechanism 23 and the dispensing mechanism 21 and 22. The dispensing mechanism enables attaching thereto and detaching therefrom dispensing chips 32 and includes a suction and discharge mechanism 22 capable of suctioning and discharging a liquid through the dispensing chip 32 and a liquid supply mechanism 21 discharging a liquid directly from a nozzle.

The arrangement in Fig. 1A and Fig. 1B is exemplification and the arrangement of the members is not limited to this. The dispensing chip/reagent setting part 11, the sample setting part 12, the warming/stirring part 13, and the nucleic acid extracting part 14 may be arranged each at an optional point only when the optional point is within a range movable by the dispensing and pressurizing unit 15.

This nucleic acid separating apparatus 10 includes the dispensing mechanism 21 and 22 that injects the specimen liquid and the reagent through the dispensing chips 32, and the pressured gas supply mechanism 23 that supplies a pressurized gas. Plural steps such as the dispensing and pressurizing can thereby be automated and not only the nucleic acid extraction step but also the traditionally manually performed pre-process steps can be automated.

The constituent members of the nucleic acid separating apparatus 10 will be described below.

### <Dispensing Chip/Reagent Setting Part>

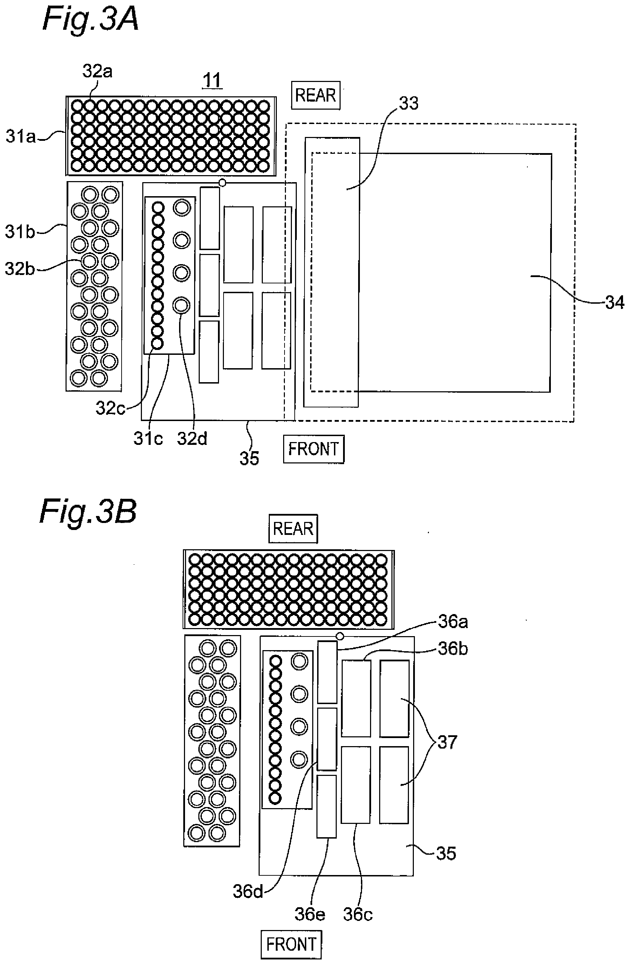

Fig. 3A is a plan diagram of the overall configuration of the dispensing chip/reagent setting part 11 of the nucleic acid separating apparatus 10 in Fig. 1. Fig. 3B is a plan diagram for explaining the configuration in a reagent container tray 35 of the dispensing chip/reagent setting part 11 of the nucleic acid separating apparatus 10 in Fig. 1.

Dispensing chips 32a, 32b, and 32c and 32d are respectively stored in dispensing chip racks 31a, 31b, and 31c. The dispensing chips 32a, 32b, 32c, and 32d may each have a capacity different from that of each other. For example, the dispensing chips 32a and 32c may each have the capacity of 1.2 ml and the dispensing chips 32b and 32d may each have the capacity of 10 ml. A dispensing chip ejecting part 33 is disposed as a point for detaching the unnecessary dispensing chip from the dispensing and pressurizing unit 15. The detached dispensing chip 32 is discarded into a dispensing chip discarding box 34. The dispensing chip discarding box 34 may be disposed in, for example, the lower portion of the apparatus.

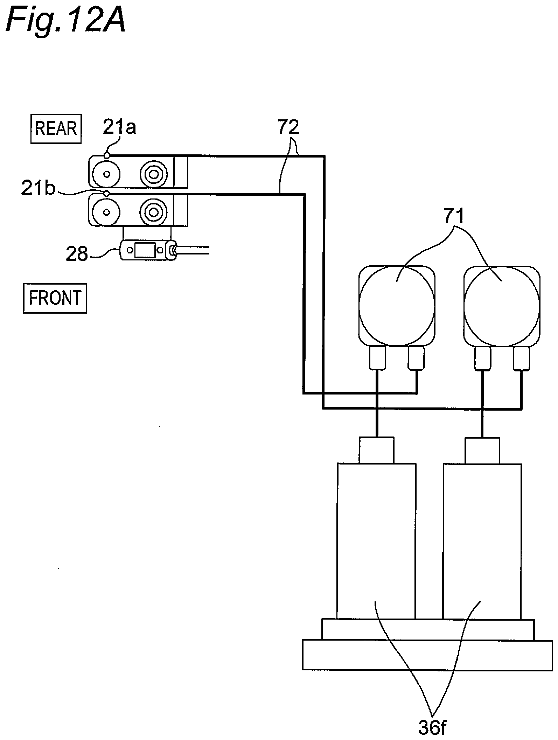

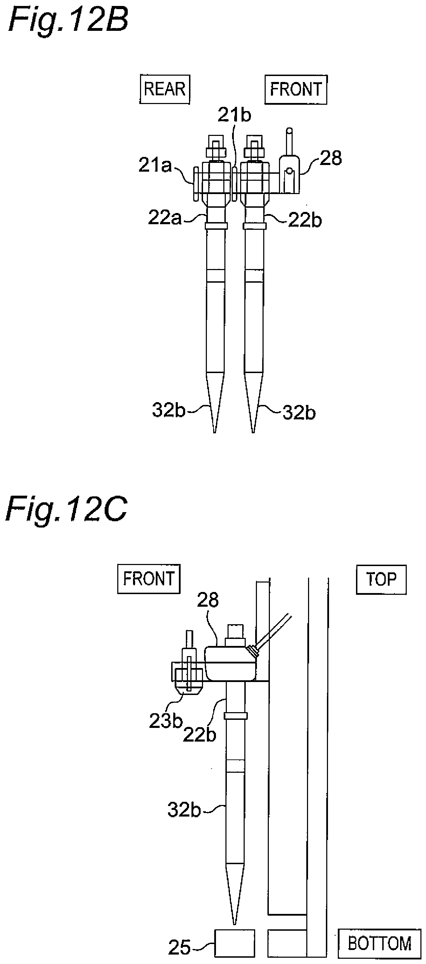

Reagent containers 36a, 36b, 36c, 36d, and 36e and waste containers 37 for discarding the reagents are disposed on the reagent container tray 35. The reagents are, for example, a pre-process enzyme, a solution liquid, and ethanol that are used at the pre-process steps and a cleaning liquid and a collection liquid that are used at the nucleic acid extracting step. For example, the pre-process enzyme may be put in the reagent container 36a, the solution liquid may be put in the reagent container 36b, ethanol may be put in the reagent container 36c, and the collection liquid may be put in the reagent container 36d. Which reagent is put in which reagent container only has to be properly set taking into consideration the convenience. A reagent bottle 36f may separately be disposed for the cleaning liquid in the lower portion of the apparatus as depicted in Fig. 12 because the cleaning liquid is used in a large amount.

### <Sample Setting Part>

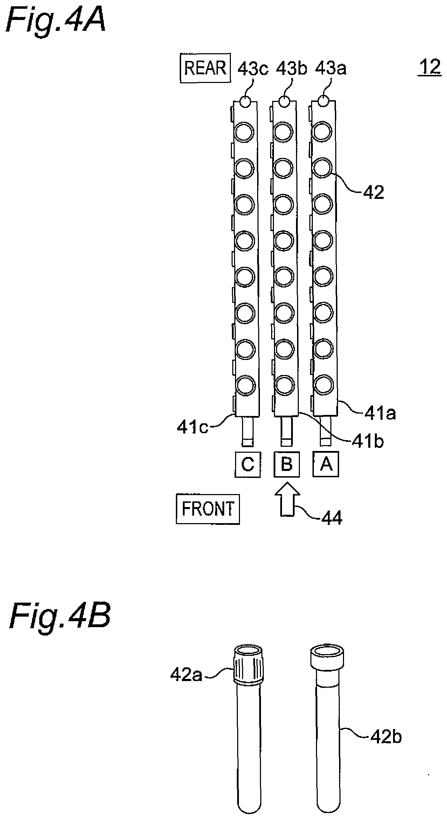

Fig. 4A is a plan diagram of the configuration of the sample setting part 12 of the nucleic acid separating apparatus 10 in Fig. 1. Fig. 4B is a diagram of an example of sample containers 42a and 42b to be arranged in the sample setting part 12 in Fig. 4A. The sample container 42 is, for example, a blood collection tube and has the specimen from which nucleic acid is extracted. The sample containers 42 are stored in sample racks 41a, 41b, and 41c. As depicted in Fig. 4A, the plural sample containers 42 are retained in rows in the sample racks 41a, 41b, and 41c. In this case, each of the sample racks stores therein eight sample containers 42. In the example in Fig. 4A, the 24 sample containers 42 as a whole can be stored.

Sample rack presence or absence sensing sensors 43a, 43b, and 43c of the sample racks 41a, 41b, and 41c are respectively disposed at the back ends in a setting direction 44 of the sample racks 41a, 41b, and 41c. The sample rack presence or absence sensing sensors 43a, 43b, and 43c respectively detect whether the sample racks 41a, 41b, and 41c are set. As to each of the sample racks 41a, 41b, and 41c, for each corresponding lane, a shape engaging with each other may be disposed in the back end portions. Each of the sample racks 41a, 41b, and 41c thereby does not engage with the non-corresponding lane at its end portion and any mistake of inserting into a wrong lane can be suppressed.

As depicted in Fig. 4B, as to the sample containers 42, the sample containers 42a and 42b having various shapes are usable.

### <Specimen>

In, for example, the diagnosis field, each of such items collected as test substances is handled as a specimen, as biological fluids such as the whole blood, blood plasma, blood serum, urine, feces, semen, and saliva, or a plant (or a portion thereof), an animal (or a portion thereof), or a melt of each thereof, and a solution prepared from a biological material such as a homogenate.

### <Warming/Stirring Part>

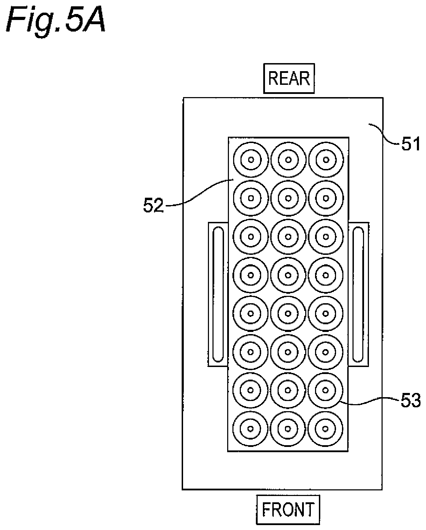

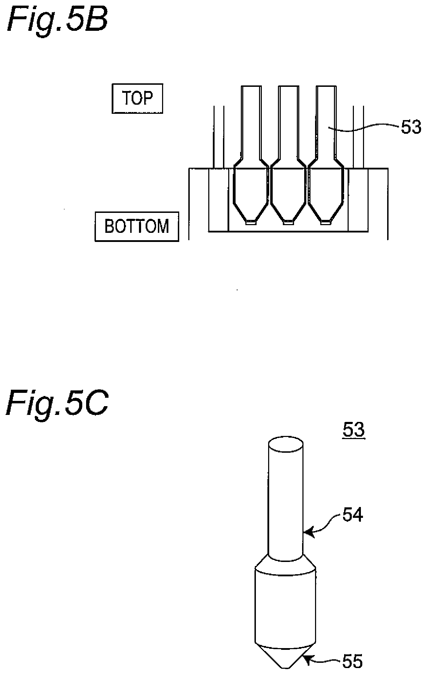

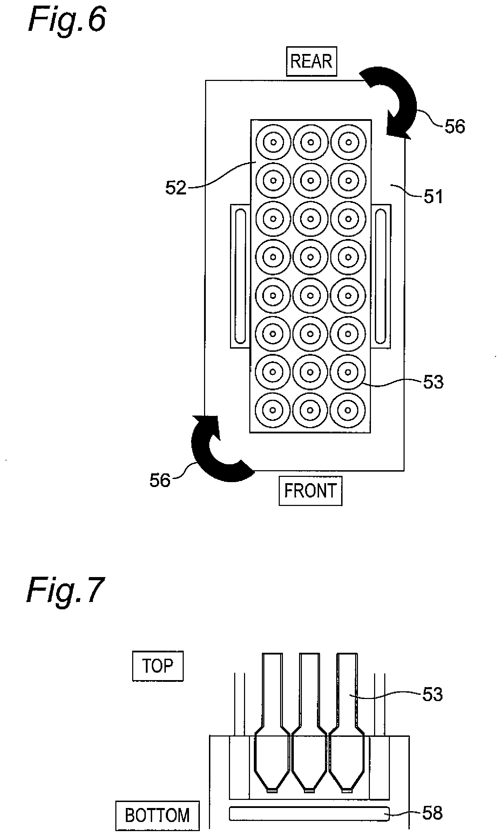

Fig. 5A is a plan diagram of the configuration of the warming/stirring part 13 of the nucleic acid separating apparatus 10 in Fig. 1. Fig. 5B is a front diagram of Fig. 5A. Fig. 5C is a perspective diagram of the structure of each of the process tubes 53 to be arranged in the warming/stirring part 13 in Fig. 5A. Fig. 6 is a plan diagram of the state where the process tubes 53 are stirred in the warming/stirring part 13. Fig. 7 is a front diagram of the warming/stirring part 13 that warms the process tubes.

The warming/stirring part 13 can stir and warm the liquid in each of the process tubes 53. A process tube rack 52 storing the process tubes 53 in the stirring unit 51 is disposed in the warming/stirring part 13. In the example in Fig. 5A, the eight process tubes 53 are lined in one row and three rows are arranged to have the 24 process tubes 53 as a whole. The process tubes 53 each correspond to the specimen in one of the sample containers 42. In each of the process tubes 53, the specimen in the corresponding sample container 42 is resolving-processed.

As depicted in Fig. 5C, the process tube 53 is characterized by including a thin neck portion 54, a thick body, and a cone-like bottom portion 55. Any flying about of a liquid caused by ascending thereof can be suppressed even during the stirring with the upper face left opened by forming a "turner" between the thin neck portion 54 and the thick body. Liquid remaining during the suction by the dispensing chip 32 can be reduced by shaping the bottom portion into a cone-like shape.

As depicted in Fig. 6, during the stirring, the stirring unit 51 causes the overall process tube rack 52 to eccentric-rotate and the specimen liquid in each of the process tubes 53 is thereby stirred. The stirring unit 51 corresponds to the stirring mechanism. For the stirring, for example, the rotation number is 1,500 rpm or smaller and the amplitude is, for example, 1 mm to 10 mm. The stirring time period is, for example, 240 seconds or shorter.

As depicted in Fig. 7, during the warming, a heater 58 in the lower portion of the process tube rack 52 can warm the process tubes 53. For the warming, for example, the temperature is 20°C to 60°C, at which the pre-process enzyme and the solution liquid excellently work.

### <Nucleic Acid Extracting Part>

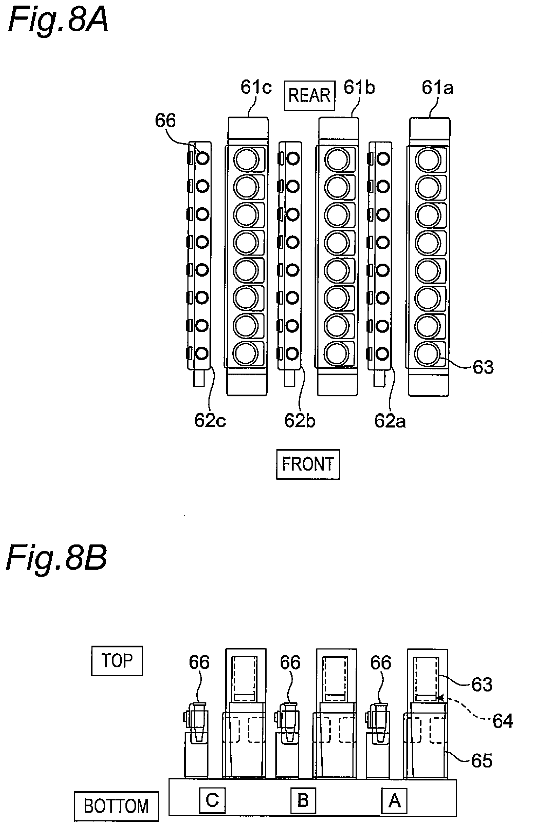

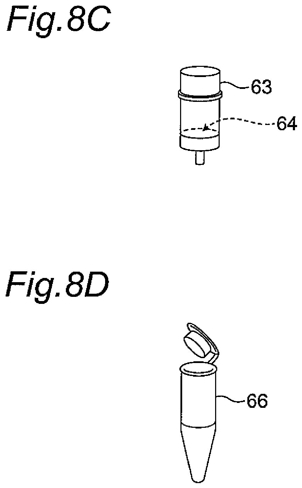

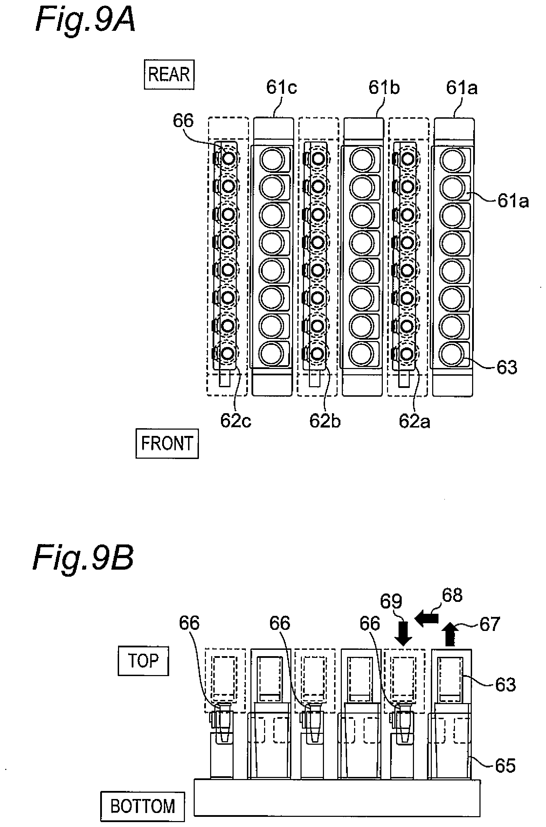

Fig. 8A is a plan diagram of the configuration of the nucleic acid extracting part 14 of the nucleic acid separating apparatus 10 in Fig. 1. Fig. 8B is a front diagram of Fig. 8A. Fig. 8C is a schematic perspective diagram of the configuration of a filter cartridge 63 to be arranged in the nucleic acid extracting part 14 in Fig. 8A. Fig. 8D is a schematic perspective diagram of the configuration of a collection tube 66 to be arranged in the nucleic acid extracting part 14 in Fig. 8A. Fig. 9A is a plan diagram of the state where the filter cartridge 63 is moved onto the collection tube 66 for collecting nucleic acid to separate nucleic acid, in the nucleic acid extracting part 14. Fig. 9B is a front diagram of Fig. 9A.

The nucleic acid extracting part 14 includes filter cartridge racks 61a, 61b, and 61c each storing therein the filter cartridges 63, and collection tube racks 62a, 62b, and 62c each storing therein the collection tubes 66. The filter cartridge racks 61a, 61b, and 61c each store therein eight filter cartridges 63. The collection tube racks 62a, 62b, and 62c each store therein eight collection tubes 66. In the example in Fig. 8A, the 24 filter cartridges 63 and the 24 collection tubes 66 are stored. One pair including the filter cartridge 63 and the collection tube 66 corresponds to the one sample container 42 and the one process tube 53. Into the collection tube 66, the specimen liquid including nucleic acid resolving-processed in the corresponding process tube 53 is dispensed. For each of the collection tubes 66, nucleic acid included in the specimen in the corresponding one sample container 42 is finally collected.

As depicted in Fig. 8A, the filter cartridge racks 61a, 61b, and 61c, and the collection tube racks 62a, 62b, and 62c are alternately arranged between each other. The filter cartridge rack 61a and the collection tube rack 62a correspond to each other. Similarly, the filter cartridge rack 61b and the collection tube rack 62b correspond to each other, and the filter cartridge rack 61c and the collection tube rack 62c correspond to each other.

The filter cartridge racks 61a, 61b, and 61c may each have the shape engaging with each other disposed at the back end portion for each corresponding lane. The filter cartridge racks 61a, 61b, and 61c each thereby do not engage in its end portion in any non-corresponding lane and any mistake of inserting into a wrong lane can therefore be suppressed. Similarly, the collection tube racks 62a, 62b, and 62c may each have the shape engaging with each other disposed at its back end portion for each corresponding lane. The collection tube racks 62a, 62b, and 62c each thereby do not engage in its end portion in any non-corresponding lane and any mistake of inserting into a wrong lane can therefore be suppressed.

As depicted in Fig. 8B, in each of the filter cartridge racks 61a, 61b, and 61c, a waste tube 65 is connected beneath the cartridge 63. On the other hand, in the collection tube racks 62a, 62b, and 62c, only the collection tubes 66 are set until the nucleic acid extraction step is executed.

### <Filter>

As depicted in Fig. 8C, the filter cartridge 63 includes a filter 64. The filter 64 (a nucleic acid adsorbing porous film) is a porous film that adsorbs nucleic acid by a mutual action that involves no ionic bonding. The filter 64 is configured to maintain the adsorption with nucleic acid during the cleaning by the cleaning liquid, and weaken the adsorption force of nucleic acid to release the nucleic acid during the collection by the collection liquid. It is more preferred that the filter 64 be a porous film including a hydroxyl group as its hydrophilic group, and that the porous film mean a porous film whose material itself is a porous film including a hydroxyl group or a porous film having a hydroxyl group introduced therein by processing or coating the material forming a porous film. The material forming the porous film may be either an organic substance or an inorganic substance while an organic material such as an organic polymer is advantageously used from the easiness of processing. The filter 64 includes a porous film including, for example, nylon, polysulfone, polyethersulfone, polycarbonate, polyacrylate, an acrylate copolymer, polyurethane, polyamide, polyvinyl chloride, polyfluorocarbonate, polytetrafluoroethylene, polyvinylidene difluoride, a polyethylene-tetrafluoroethylene copolymer salt, polybenzimidazole, a polyethylene-chrolotrifluoroethylene copolymer salt, plyimide, polyphenylene sulfide, cellulose, cellulose-mixed ester, nitrocellulose, acetylcellulose, polyacrylonitrile, a polyacrylonitrile copolymer, nitrocellulose, polypropylene, and/or polyester.

Examples of the porous film of an organic material including a hydroxyl group include a surface-saponified substance of acetylcellulose described in Japanese Laid-Open Patent Publication No. 2003-128691. This acetylcellulose may be any one of monoacetylcellulose, diacetylcellulose, and triacetylcellulose while triacetylcellulose is especially advantageously used. In this case, the amount (the density) of hydroxyl groups on the solid phase surface can be controlled using the degree of the saponification process (saponification degree). To improve the separation efficiency of nucleic acid, it is preferred that the amount (the density) of hydroxyl groups be large. For example, in the case of acetylcellulose such as triacetylcellulose, the saponification ratio is preferably 5% or higher and further preferably 10% or higher. To increase the surface area, a saponification process is applied to the porous film of acetylcellulose. The spatial amount (the density) of hydroxyl groups can be controlled by a combination of the degree of the saponification process (the saponification degree) and the pore diameter of the porous film. In this case, the porous film may be a porous film that is symmetrical for its front side and its back side while a porous film that is not symmetrical for its front side and its back side can advantageously be used.

In addition, examples of the porous film of the organic material including a hydroxyl group include porous films each formed by polyhydroxyethyl acrylic acid, polyhydroxyethyl methacrylic acid, polyvinyl alcohol, polyvinyl pyrrolidone, polyacrylic acid, polymethacrylic acid, polyoxyethylene, acetylcellulose, a mixture of acetylcelluloses each having an acetyl value different from that of each other, or the like while a porous film of an organic material that has a polysaccharide structure can advantageously be used. Especially, the porous film of an organic polymer including a mixture of acetylcelluloses each having an acetyl value different from that of each other can advantageously be used, and such mixtures can advantageously be used as a mixture of triacetylcellulose and diacetylcellulose, a mixture of triacetylcellulose and monoacetylcellulose, a mixture of triacetylcellulose, diacetylcellulose, and monoacetylcellulose, and a mixture of diacetylcellulose and monoacetylcellulose.

Examples of the filter 64 include a porous film including an organic material formed by applying a saponification process to a mixture of acetylcelluloses each having an acetyl value different from that of each other and, for example, an oxide of each of the above mixtures of acetylcelluloses can advantageously be used. The saponification process is conducted by bringing acetylcellulose into contact with a saponification process liquid (such as, for example, sodium hydroxide), and a portion of acetylcellulose in contact with the saponification process liquid becomes regenerated cellulose and hydroxyl groups are introduced thereinto. To improve the separation efficiency of nucleic acid, it is more advantageous to introduce a larger number of hydroxyl groups. For example, the saponification ratio is preferably about 5% or higher and more preferably about 10% or higher.

A porous film of regenerated cellulose can advantageously be used as the filter 64. The "regenerated cellulose" is formed by converting the surface or the whole of solid acetylcellulose into cellulose by applying a saponification process thereto, and is different from the original cellulose in the points of the crystal condition and the like.

Other examples of the filter 64 include a porous film including a silica compound as a porous film of an inorganic material including a hydrophilic group such as, for example, a glass filter. Other examples thereof include a porous silica thin film as described in Japanese Patent Publication No. 3058342. This porous silica thin film can be produced by spreading out on a substrate a developing solvent of a cation-type amphiphilic substance having a bimolecular membrane forming function, thereafter removing the solvent from the liquid film on the substrate, thereby preparing a multi-layer bimolecular membrane thin film of the amphiphilic substance, bringing this film into contact with a solution including a silica compound, and removing by extracting the multi-layer bimolecular membrane thin film.

A porous film is usable as the filter 64, whose thickness is 10 µm to 500 µm and preferably 50 µm to 250 µm. A porous film is usable whose minimal pore diameter is 0.22 µm or larger and more preferably 0.5 µm or larger. A porous film is usable whose ratio of its maximal pore diameter to its minimal pore diameter is 2 or greater and further preferably 5 or greater. A porous film is usable whose void ratio is 50% to 95% and further preferably 65% to 80%.

The one filter 64 may be used while the plural filters 64 may also be used. The plural filters 64 may be same ones or may be different ones. The filters 64 may be a combination of an inorganic material filter(s) and an organic material filter(s). Examples thereof include, for example, a combination of a glass filter and a regenerated cellulose porous film. The plural filters may be a combination of an inorganic material filter(s) and an organic material porous film(s) having no adsorptive property for nucleic acid. Examples thereof include, for example, a combination of a glass filter(s) and a porous film(s) of nylon or polysulfone.

### <Collection Tube>

As depicted in Fig. 8D, the collection tube 66 may have a lid disposed thereon that can close the upper face. Nucleic acid is collected in the collection tube 66 together with the collection liquid.

### <About Moving of Filter Cartridge>

As depicted in Fig. 9B, the filter cartridges 63 need to be moved from the filter cartridge racks 61a, 61b, and 61c to the collection tube racks 62a, 62b, and 62c before the collection of nucleic acid.

For example, the filter cartridges 63 are moved in the following three stages.
1) The filter cartridges 63 are first moved to be lifted by the filter cartridge racks 61a, 61b, and 61c.
2) The filter cartridges 63 are next horizontally moved from the position above the filter cartridge racks 61a, 61b, and 61c to a position above the collection tube racks 62a, 62b, and 62c.
3) The filter cartridges 63 are next moved to be descended from the position above the collection tube racks 62a, 62b, and 62c.

The filter cartridges 63 can be moved from the filter cartridge racks 61a, 61b, and 61c to the collection tube racks 62a, 62b, and 62c by the above operations. The filter cartridges 63 may be fixed and the collection tubes 66 and the waist tubes 65 may be moved.

### <Dispensing and Pressurizing Unit>

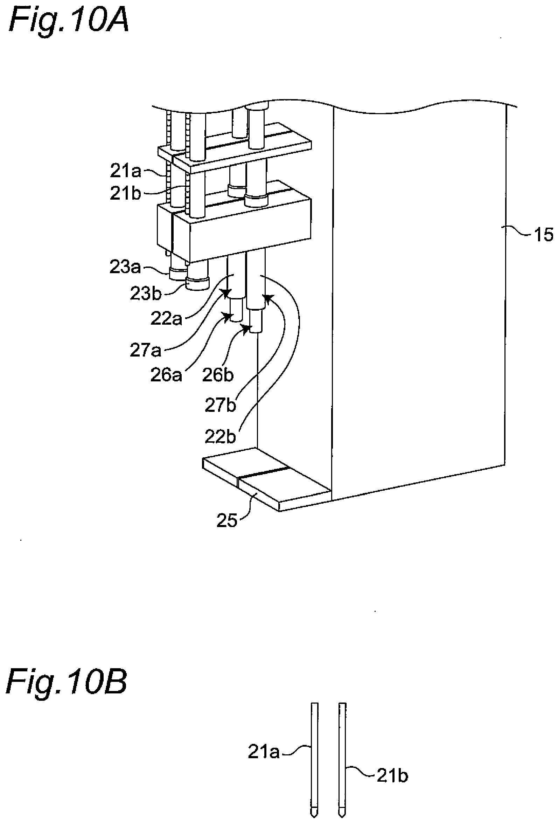

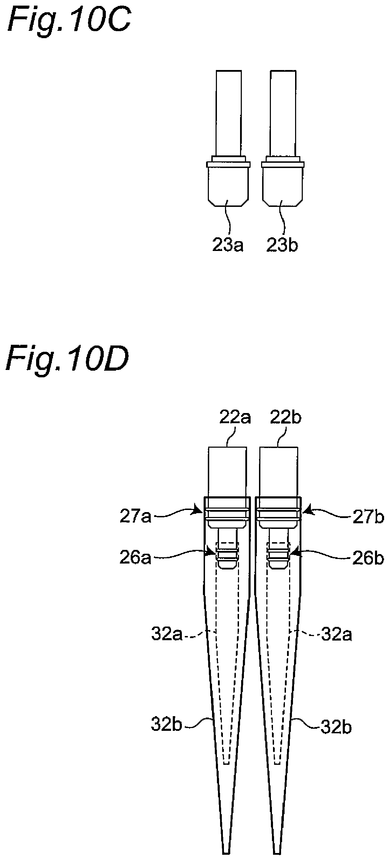

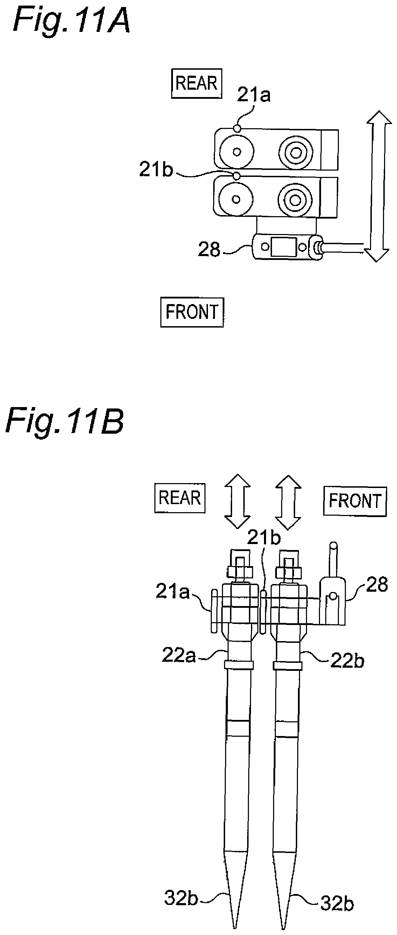

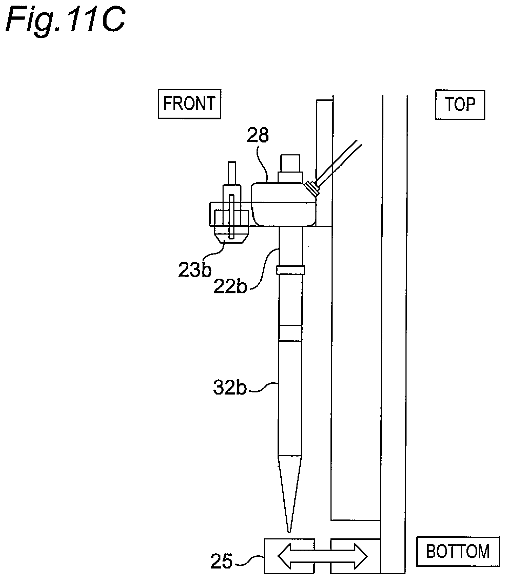

Fig. 10A is a schematic perspective diagram of the configuration of the dispensing and pressurizing unit 15 of the nucleic acid separating apparatus 10 in Fig. 1. Fig. 10B is a partial diagram of liquid supply nozzles 21a and 21b of the dispensing and pressurizing unit 15 in Fig. 10A. Fig. 10C is a partial diagram of pressurizing heads 23a and 23b of the dispensing and pressurizing unit 15 in Fig. 10A. Fig. 10D is a partial diagram of suction/discharge nozzles 22a and 22b of the dispensing and pressurizing unit 15 in Fig. 10A. Fig. 11A is a plan diagram of the dispensing and pressurizing unit 15. Fig. 11B is a side diagram of the dispensing and pressurizing unit 15. Fig. 11C is a front diagram of the dispensing and pressurizing unit 15.

The dispensing and pressurizing unit 15 includes dispensing mechanisms 21a, 21b, 22a, and 22b that each supply a liquid for containers whose upper face is open in the vertical direction (the process tubes 53 and the filter cartridges 63), pressurized gas supply mechanisms 23a and 23b that each supply a pressurized gas into the containers 63, and a move mechanism 24 that moves the overall body to a position above the containers 53 and 63 in the vertical direction. The dispensing mechanism includes a suction and discharge mechanism 22a and 22b, and liquid supply mechanisms 21a and 21b that each directly discharge a liquid from a nozzle. The suction and discharge mechanism 22a and 22b is capable of attaching thereto and detaching therefrom dispensing chips 32a and 32b. The suction and discharge mechanism 22a and 22b discharges a liquid such as a specimen liquid or a reagent through the dispensing chips 32a and 32b to the containers 53 and 63, and suction a liquid from the containers 53 to the dispensing chips 32a and 32b. The liquid supply mechanisms 21a and 21b can each supply a liquid to the containers 53 and 63.

The dispensing and pressurizing unit 15 may have a work sensing sensor 28 disposed therein. The work sensing sensor 28 senses whether the sample containers 42, the dispensing chips 32, the reagent containers 36, the process tubes 53, the filter cartridges 63, the collection tubes 66, and the like are each set at the predetermined positions for the predetermined quantity. A photoelectric sensor formed by combining a CMOS sensor and a laser light source, or the like is usable as the work sensing sensor 28. The work sensing sensor 28 can be moved by the move mechanism 24 to the vicinity of a position above each of the works to be sensed and can sense presence or absence of the work. It is preferred that the work sensing sensor check each of the works before the start of the pre-process steps.

The dispensing and pressurizing unit 15 includes two sets of dispensing mechanisms 21a, 21b, 22a, and 22b, and that of pressurized gas supply mechanism 23a and 23b while the quantity is not limited to two sets and one set may be included. Otherwise, three or more sets may be included. The dispensing mechanisms 21a and 22a and the pressurized gas supply mechanism 23a, and the dispensing mechanisms 21b and 22b, and the pressurized gas supply mechanism 23b may be adapted to each be movable in a z-axis direction separately from each other. Each individual dispensing operation is enabled by the fact that the dispensing mechanisms and the like are each independently movable in the z-axis direction as above. For example, when the two sets of dispensing mechanisms are present and an odd number of sample containers 42 to be processed are present, the dispensing can first be executed for two sets thereof at one time, and the dispensing from the finally remaining one sample container can be executed by operating only one dispensing mechanism. When the dispensing mechanisms are each not independent, complicated processing is necessary for attachment of the dispensing chips, and the like because all the dispensing mechanisms similarly move.

As depicted in Fig. 10A, a receiving tray 25 may be disposed in the dispensing and pressurizing unit. The receiving tray 25 receives a liquid such as the specimen or the reagent dropping from the tip of the dispensing chip 32 when the dispensing and pressurizing unit 15 moves. The receiving tray 25 is movable in the x-axis direction and, during the dispensing operation by the dispensing mechanism 22, is accommodated at a position at which the receiving tray 25 does not interfere the dispensing operation (see Fig. 11C).

The members constituting the dispensing and pressurizing unit 15 may each be disposed separately from each other without constituting the one unit.

### <Suction and Discharge Mechanism>

The suction and discharge mechanism (a pipette) 22a and 22b is characterized in that the mechanism does not dispense the cleaning liquid and the like each through a pipe directly connected to a bottle including the cleaning liquid or the like but each inject the liquid through the dispensing chip 32 attached to the tip thereof. A problem is traditionally present that many dispensing mechanisms need to be prepared each for a type of liquid such as the cleaning liquid or the collection liquid. A method is present according to which one discharge nozzle is set at the tip and the liquid to be discharged is switched by a switching valve or the like. In this case, however, to avoid any mixing of the liquids during the switching, cleaning using the liquid to be used after the switching, or the like needs to be executed and discarding of the wasted liquid occurs. In contrast, according to the suction and discharge mechanism 22a and 22b, dispensing can be executed changing the dispensing chip 32 for each of the liquids to be dispensed. The substantially one set of suction and discharge mechanism 22a and 22b can dispense various liquids. Any gas in the dispensing chip 32 can be suctioned or discharged, and a liquid can be suctioned and discharged in the dispensing chip 32.

As depicted in Fig. 10D, the suction and discharge mechanism 22a and 22b may respectively include, for example, first dispensing chip attaching parts 26a and 26b each having an O-ring having a first caliber at the tip thereof and capable of attaching thereto and detaching therefrom the first dispensing chip 32a, and second dispensing chip attaching parts 27a and 27b each having an O-ring having a second caliber larger than the first caliber on the inner side of the tip thereof and capable of attaching thereto and detaching therefrom the second dispensing chip 32b. The one suction and discharge mechanism 22a and 22b can thereby attach the two types of dispensing chip 32a and 32b each having a capacity different from that of each other. The proper dispensing chip can thereby be used matching with the dispensing amount.

### <Liquid Supply Mechanism>

Fig. 12A is a plan diagram of liquid supply nozzles 21a and 21b that each discharge a liquid in the dispensing and pressurizing unit 15, and tube pumps 71 and reagent bottles 36f connected to the liquid supply nozzles (the liquid supply mechanisms) 21a and 21b. Fig. 12B is a side diagram of the dispensing and pressurizing unit 15. Fig. 12C is a front diagram of the dispensing and pressurizing unit 15. The liquid supply nozzles 21a and 21b each supply a reagent (such as, for example, a cleaning liquid) from the reagent bottle 36f by the tube pump 71. The supply amount for one session by each of the liquid supply nozzles 21a and 21b is, for example, 12 ml or smaller and the liquid may be supplied in units each of 0.25 ml.

### <Pressurizing Head (Pressurized Gas Supply Mechanism)>

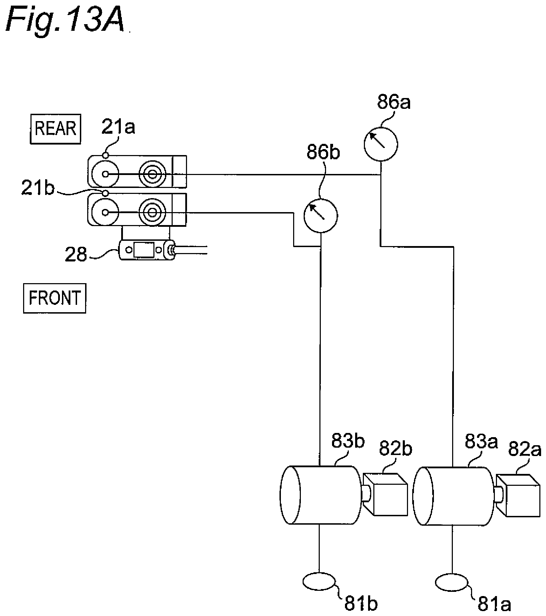

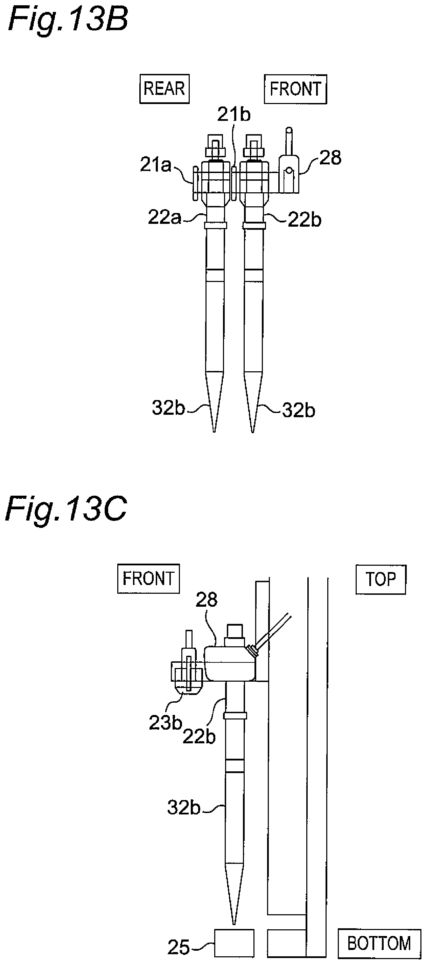

Fig. 13A is a plan diagram of the pressurizing heads 23a and 23b in the dispensing and pressurizing unit 15, and pipes connected to the pressurizing heads 23a and 23b. Fig. 13B is a side diagram of the dispensing and pressurizing unit 15. Fig. 13C is a front diagram of the dispensing and pressurizing unit 15.

The pressurizing heads 23a and 23b are respectively connected to air filters 81a and 81b that are disposed at a ventilating opening through pulse motors 82a and 82b, pressurizing pumps 83a and 83b, and pressure sensors 86a and 86b. The set range of the pressure is, for example, 30 kPa to 160 kPa. For example, air or nitrogen is usable as the pressurized gas.

The pressurizing heads 23a and 23b are arranged to avoid their interference on the operations of the suction and discharge mechanism 22a and 22b and the liquid supply nozzles 21a and 21b.

### <Move Mechanism>

The move mechanism 24 can move the overall dispensing and pressurizing unit 15 to the position above the containers 53 and 63 in the vertical direction. For example, as depicted in Fig. 1A and Fig. 1B, an X-axis actuator 24a, a Y-axis actuator 24b, and a Z-axis actuator 24c are suable as the move mechanism 24.

### <Control Unit>

The control unit 16 controls the dispensing chip/reagent setting part 11, the sample setting part 12, the warming/stirring part 13, the nucleic acid extracting part 14, and the dispensing and pressurizing unit 15 that constitute the nucleic acid separating apparatus 10. The control part 16 may be, for example, a computer. The computer only has to include the physical components such as, for example, a CPU 1, a memory 2, a storing part 3, a displaying part 4, an input part 5, and an interface 6.

### <Nucleic Acid Separation Method>

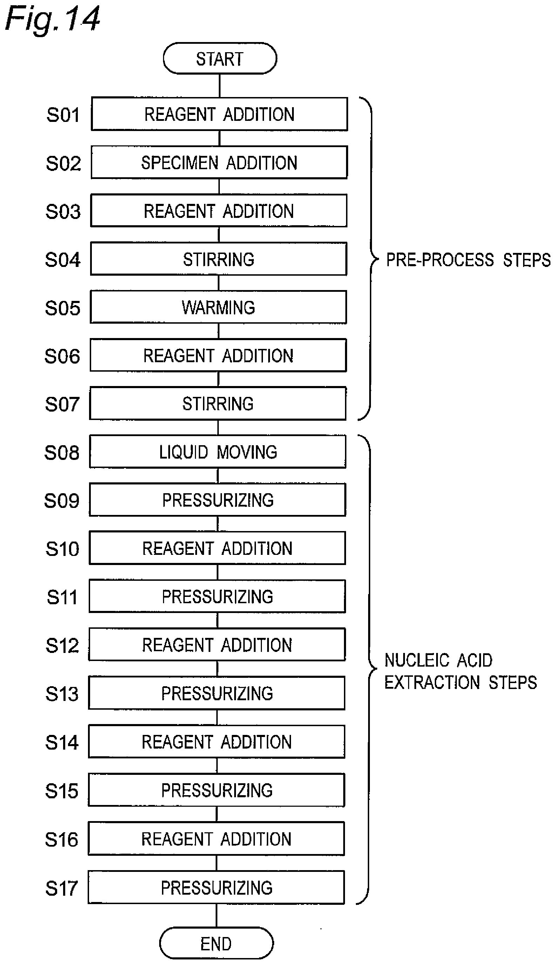

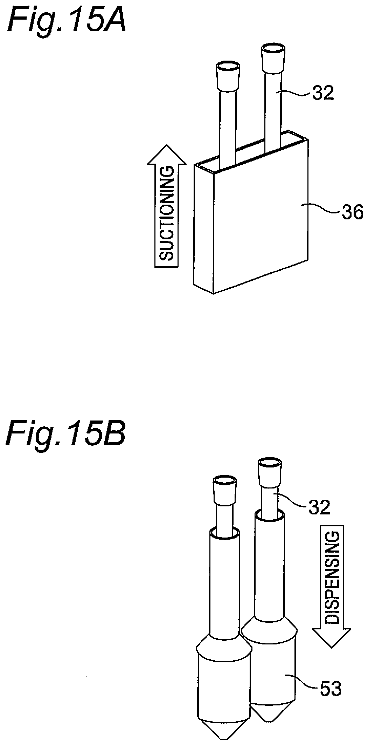

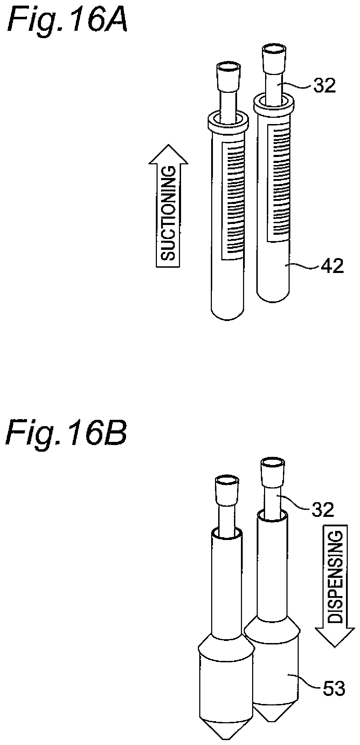

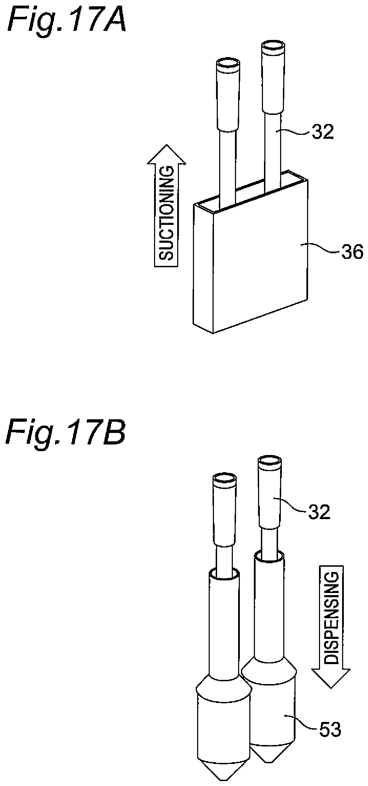

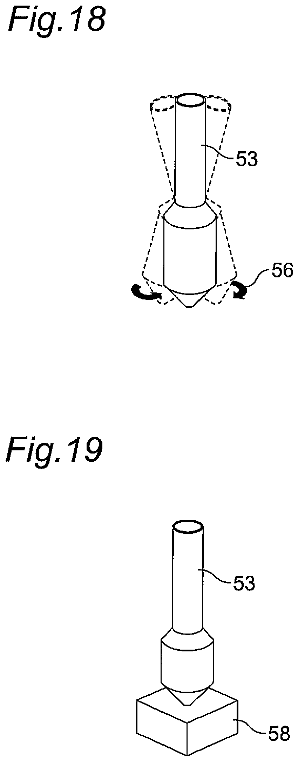

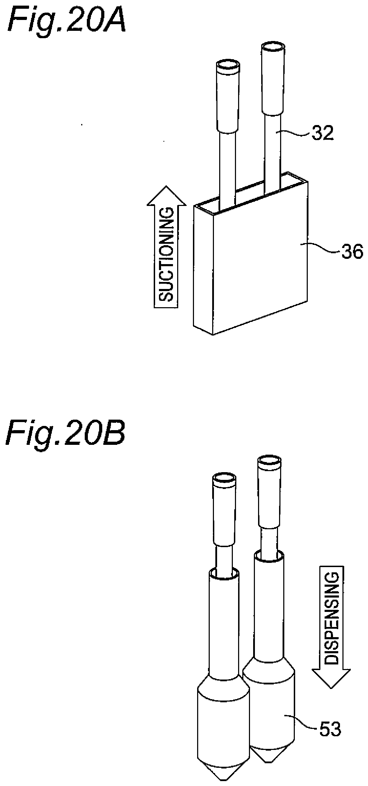

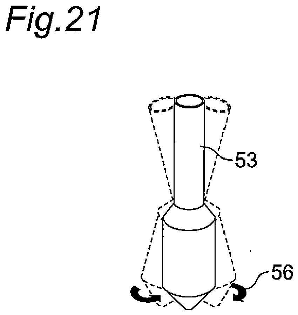

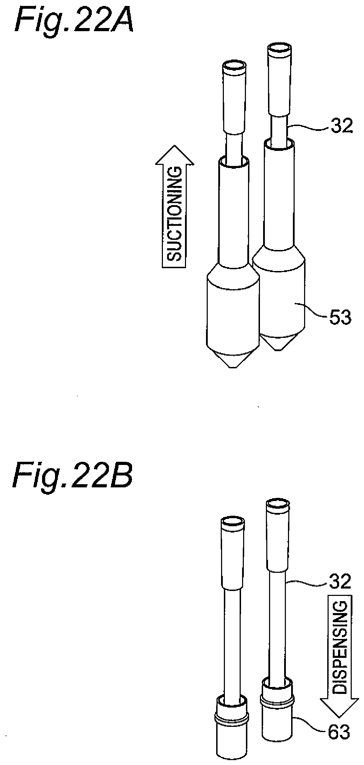

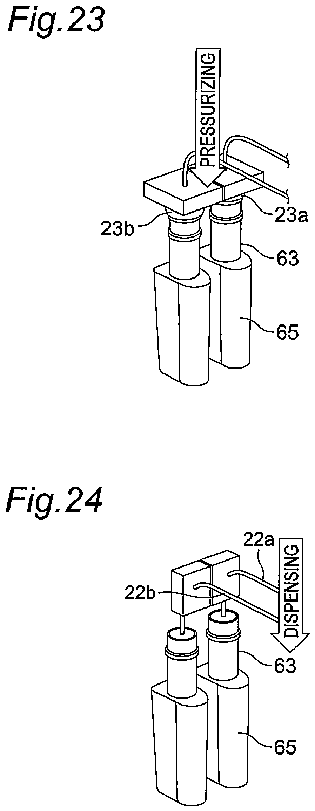

Fig. 14 is a flowchart of steps of a nucleic acid separation method according to the first embodiment. In Fig. 14, steps from a reagent addition step (S01) to a stirring step (S07) are the pre-process (the resolving process) steps and steps from a liquid moving step (S08) to a pressurizing step (S17) are the nucleic acid extraction (the separation and collection) steps.
(1) The reagent (a pre-process enzyme) is suctioned from the reagent container 36 to the dispensing chip 32 (Fig. 15A) and is dispensed into the process tube 53 (Fig. 15B) (the reagent addition step S01).
(2) The specimen (the whole blood) is suctioned from the sample container (a blood collection tube, 42) to the dispensing chip 32 (Fig. 16A) and is dispensed into the process tube 53 (Fig. 15B) (a specimen addition step S02).
(3) The reagent (the solution liquid) is suctioned from the reagent container 36 to the dispensing chip 32 (Fig. 17A) and is dispensed into the process tube 53 (Fig. 17B) (a reagent addition step S03).
(4) The process tube 53 is stirred (Fig. 18) (a stirring step S04). The stirring of the process tube 53 is executed at the rotation number of the motor of 1,500 rpm or smaller. The amplitude is 1 mm to 10 mm.
(5) The process tube 53 is warmed (Fig. 19) (a warming step S05). The warming is executed in, for example, a temperature range from 20°C to 50°C.
   The "resolving process" is processing executed using a water solution including a reagent (such as a solution including, for example, chaotropic salt or guanidine salt, a surfactant, and a protein-degrading enzyme) that resolves the cell membrane and the nucleic membrane and thereby causes the nucleic acid to be soluble and, for example, when the specimen to be processed is the whole blood, the red blood cells and various types of protein are degraded to be low molecules to prevent any non-specific adsorption to and any clogging of the filter 64, and the white blood cells and the nucleic membranes are resolved to cause the nucleic acid to be extracted, to be soluble. Examples of a "water-soluble organic solvent" include ethanol, isopropanol, propanol, or the like and, among these, ethanol is advantageously used. The concentration of the water-soluble organic solvent is preferably 5% by weight to 90% by weight and further preferably 20% by weight to 60% by weight. The addition concentration of ethanol is especially advantageously set to be as high as possible to the extent that no aggregation is formed.
   The whole blood, or a test substance including cells or viruses is resolving-processed and this produces the state where the water-soluble organic solvent is added to a solution that has nucleic acid dispersed in the liquid.
(6) The reagent (ethanol) is suctioned from the reagent container 36 to the dispensing chip 32 (Fig. 20A) and is dispensed to the process tube 53 (Fig. 20B) (a reagent addition step S06).
(7) The process tube 53 is stirred (Fig. 21) (the stirring step S07). The stirring of the process tube 53 is executed at the rotation number of the motor of 1,500 rpm or smaller. The amplitude thereof is 1 mm to 10 mm.
(8) The specimen liquid including resolving-processed nucleic acid is suctioned from the process tube 53 to the dispensing chip 32 (Fig. 22A) and is dispensed to the filter cartridge 63 (Fig. 22B) (the liquid move step S08). The specimen liquid including resolving-processed nucleic acid can thereby be injected into the filter cartridge 63.
(9) The filter cartridge 63 is filtered under pressure (Fig. 23) (a pressurizing step S09). The filtering under pressure is executed by introducing a pressurized air into the filter cartridge 63 to pressurize its inside. In this case, the inside of the filter cartridge 63 is maintained to be in a sealed state. The specimen liquid is thereby caused to pass through the filter 64 and nucleic acid is adsorbed by the filter 64. The liquid-state portion passing through the filter 64 is discharged through the waste tube 65 arranged under the filter cartridge 63. When the overall specimen liquid passes through the filter 64, the pressure is lowered to a liquid discharge completion pressure or lower, and the end of the extractoin is detected by the pressure sensors 86a and 86b at each of all the filter cartridges 63. For example, the pressurizing heads 23a and 23b are thereafter moved to be lifted, the sealed state is cancelled, and the pressurizing step comes to an end.
(10) The reagent (the cleaning liquid) is dispensed from the liquid supply nozzles 21a and 21b to the filter cartridge 63 (Fig. 24) (a reagent addition step S10).

### <Cleaning Liquid>

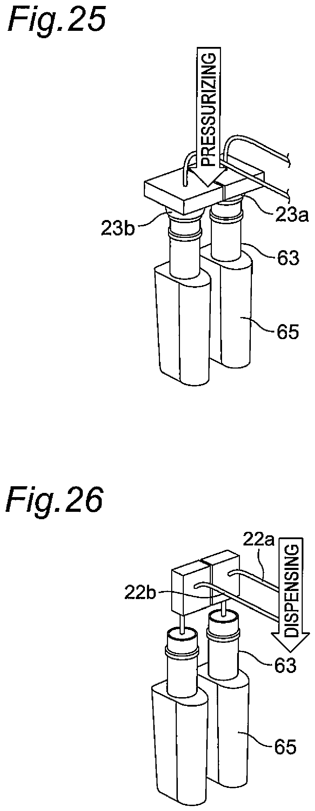

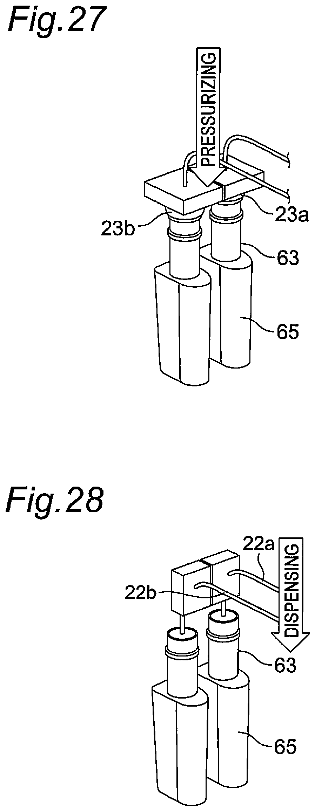

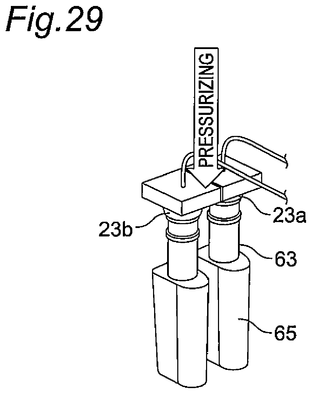

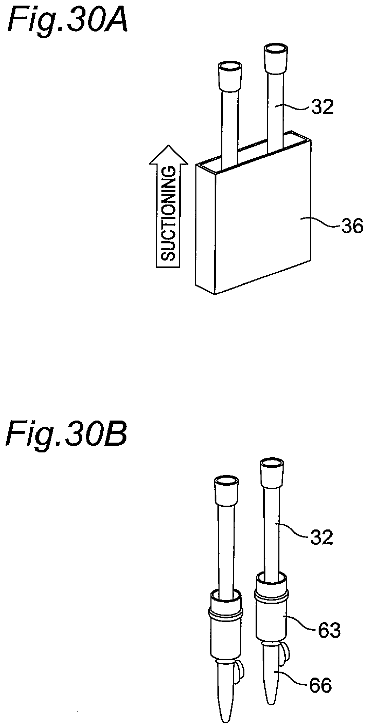

The "cleaning liquid" has a function of washing away any impurity in the specimen liquid, adhering to the filter 64 together with nucleic acid, and has a composition for any impurity to depart leaving the adsorption of nucleic acid as it is. The "cleaning liquid" includes, for example, a base agent and a buffer agent, and a water solution when necessary that includes a surfactant. The main agent is methanol, ethanol, isopropanol, n-isopropanol, butanol, acetone, or the like. In the water solution, the main agent is about 10% by weight to 100% by weight, preferably 20% by weight to 100% by weight, and further preferably 40% by weight to 80% by weight in the water solution.
(11) The filter cartridge 63 is filtered under pressure (Fig. 25) (a pressurizing step S11). The filtering under pressure is executed by introducing a pressurized air into the filter cartridge 63 to pressurize its inside. The impurities other than nucleic acid are cleaned to be removed causing the filter 64 to retain nucleic acid as it is. The liquid-state portion passing through the filter 64 is discharged through the waste tube 65 connected to the filter cartridge 63.
(12) The reagent (the cleaning liquid) is dispensed from each of the liquid supply nozzles 21a and 21b to the filter cartridge 63 (Fig. 26) (a reagent addition step S12).
(13) The filter cartridge 63 is filtered under pressure (Fig. 27) (a pressurizing step S13).
(14) The reagent (the cleaning liquid) is dispensed from each of the liquid supply nozzles 21a and 21b to the filter cartridge 63 (Fig. 28) (a reagent addition step S14).
(15) The filter cartridge 63 is filtered under pressure (Fig. 29) (a pressurizing step S15). The combination of the reagent addition step S10 and the pressurizing step S11, that of the reagent addition step S12 and the pressurizing step S13, and that of the reagent addition step S14 and the pressurizing step S15 are repeated for three times in the above. The repetition is not limited to the above example and at least one combination of the reagent addition step and the pressurizing step only has to be executed.
(16) The filter cartridges 63 are moved from the filter cartridge racks 61a, 61b, and 61c to the collection tube racks 62a, 62b, and 62c (Fig. 9A and Fig. 9B). For example, as indicated by an arrow 67 in Fig. 9B, the filter cartridge 63 is moved upward. As indicated by an arrow 68 therein, the filter cartridge 63 is next horizontally moved to the position above the collection tube 66. As indicated by an arrow 69 therein, the filter cartridge 63 is next moved downward, and the filter cartridge 63 is arranged on the collection tubes 66. The filter cartridges 63 are similarly moved sequentially and the filter cartridges 63 are each arranged on one of the collection tubes 66.
(17) The reagent (the collection liquid) is suctioned from the reagent container 36 to the dispensing chip 32 (Fig. 30A) and is dispensed to the filter cartridge 63 (Fig. 30B) (a reagent addition step S16).

### <Collection Liquid>

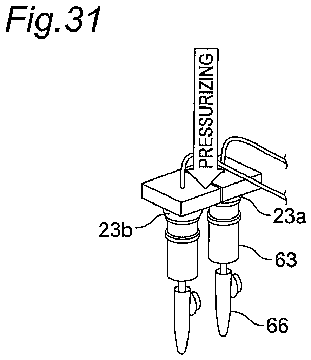

The "collection liquid" advantageously has a low salt concentration. Especially, a solution whose salt concentration is 0.5 M or lower such as, for example, purified distilled water or a TE buffer is used. "pH" of the collection liquid is preferably pH2 to pH11 and further preferably pH5 to pH9.
(18) The filter cartridge 63 is filtered under pressure (Fig. 31) (the pressurizing step S17). The filtering under pressure is executed by introducing a pressurized air into the filter cartridge 63 to pressurize its inside. The bonding force between the filter 64 and nucleic acid can be weakened by the collection liquid to cause nucleic acid adsorbed by the filter 64 to leave therefrom, and nucleic acid can be collected into the collection tube 66 together with the collection liquid. The collection tube 66 may thereafter be closed by a lid when necessary.

The collection liquid including nucleic acid can be collected by the above operation. For nucleic acid in the collection tube 66, a nucleic acid analysis process and the like are next executed.

According to the nucleic acid separating apparatus 10 and the nucleic acid separation method, for the plural sample containers 42, steps from the pre-process steps to the nucleic acid extraction step are sequentially and continuously executed for each of the sample containers. The manually executed traditional pre-process steps therefore do not need to be executed.

In this embodiment, plural sets of the sample containers 42, the process tubes 53, and the filter cartridges 63 are included while the number of sets is not limited to these. For example, the nucleic acid separating apparatus 10 according to the first embodiment is applicable to one set of the sample container 42, the process tube 53, and the filter cartridge 63.

The nucleic acid separating apparatus 10 may include a UV applying part (not depicted) that disinfects by applying a UV light beam to the inside of the apparatus. The UV applying part may be configured to apply a UV light beam moving inside the apparatus in, for example, the x- and the y-directions. The inside of the apparatus can thereby be maintained to be clean.

### (Second Embodiment)

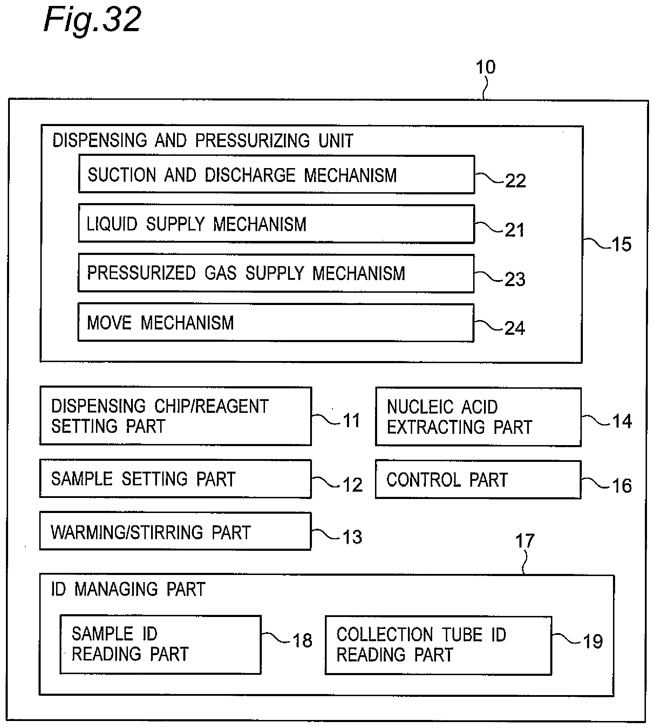

The planar configuration of a nucleic acid separating apparatus 10 according to the second embodiment seen through a top board thereof, and the configuration of the nucleic acid separating apparatus 10 seen through a front panel thereof are substantially similar to those of the first embodiment. For example, the configurations are as depicted in Fig. 1A and Fig. 1B. Fig. 32 is a block diagram of the configuration of the nucleic acid separating apparatus 10 according to the second embodiment.

The nucleic acid separating apparatus 10 includes the dispensing chip/reagent setting part 11, the sample setting part 12, the warming/stirring part 13, the nucleic acid extracting part 14, the dispensing and pressurizing unit 15, the control part 16, and an ID managing part 17. The ID managing part 17 includes a sample ID reading part 18 and a collection tube ID reading part 19. The sample ID reading part 18 reads a sample ID of a sample container 42. The collection tube ID reading part 19 reads a collection tube ID of a collection tube. The ID managing part 17 manages the sample ID and the corresponding collection tube ID correlating these with each other.

According to the nucleic acid separating apparatus 10, the ID managing part 17 can manage the sample ID of the sample container 42 and the collection tube ID of the corresponding collection tube 66 correlating these with each other. Any mistaken recognition for the sample container 42 and the corresponding collection tube 66 can thereby be suppressed.

As depicted in Fig. 1A, Fig. 1B, and Fig. 32, the nucleic acid separating apparatus 10 includes the stirring mechanism 51 that stirs and resolving-processes the process tube 53 after the specimen is injected from the sample container 42 into the process tube and the various reagents are injected thereinto, the pressurized gas supply mechanism 23 that supplies the pressurized gas to the filter cartridge 63 into which the specimen liquid is injected to separate and collect nucleic acid from the specimen liquid including nucleic acid after the resolving process to the collection tube 66, the dispensing mechanisms 21 and 22 that each inject the specimen liquid and the reagent into the process tube 53 and the filter cartridge 63, and the move mechanism 24 that moves the pressured gas supply mechanism 23 and the dispensing mechanism 21 and 22. The dispensing mechanism is able to attach thereto and detach therefrom the dispensing chip 32, and includes the suction and discharge mechanism 22 capable of suctioning and discharging a liquid through the dispensing chip 32 and the liquid supply mechanism 21 that discharges a liquid from the nozzle.

The arrangement in Fig. 1A and Fig. 1B is exemplification and the arrangement of the members is not limited to this. The dispensing chip/reagent setting part 11, the sample setting part 12, the warming/stirring part 13, and the nucleic acid extracting part 14 may each be arranged at an optional point when the optional point is in the range for the dispensing and pressurizing unit 15 to be movable.

The nucleic acid separating apparatus 10 includes the dispensing mechanism 21 and 22 that injects the specimen liquid and the reagent through the dispensing chip 32, and the pressurized gas supply mechanism 23 that supplies the pressurized gas. The plural steps such as the dispensing and the pressurizing can thereby be automated, and not only the nucleic acid extraction step but also the traditionally manually executed pre-process steps can be automated.

The constituent members of the nucleic acid separating apparatus 10 will be described below. The configurations substantially same as those of the nucleic acid separating apparatus according to the first embodiment will properly not be again described.

### <Dispensing Chip/Reagent Setting Part>

The overall configuration of the dispensing chip/reagent setting part 11 of the nucleic acid separating apparatus 10 is similar to that in the plan diagram of Fig. 3A. The configuration in the reagent container tray 35 of the dispensing/reagent setting part 11 of the nucleic acid separating apparatus 10 is similar to that in the plan diagram of Fig. 3B.

The dispensing chips 32a, 32b, and 32c and 32d are respectively stored in the dispensing chip racks 31a, 31b, and 31c. The dispensing chips 32a, 32b, 32c, and 32d, the reagent containers 36a, 36b, 36c, 36d, and 36e, the waste container 37 for discarding the reagents, and the reagents are substantially similar to those of the first embodiment and will not again be described.

### <Sample Setting Part>

The configuration of the sample setting part 12 of the nucleic acid separating apparatus 10 is similar to that in the planar diagram of Fig. 4A. The examples of the sample containers 42a and 42b arranged in the sample setting part 12 are similar to those in Fig. 4B. The sample container 42 is, for example, a blood collection tube and retains therein a specimen from which nucleic acid is extracted. The sample containers 42 are stored in the sample racks 41a, 41b, and 41c. As depicted in Fig. 4A, the plural sample containers 42 are retained in rows in the sample racks 41a, 41b, and 41c. In this case, eight sample containers 42 are retained in each of the sample racks. In the example in Fig. 4A, the 24 sample containers 42 as a whole can be stored.

The sample racks 41a, 41b, and 41c, and the sample containers 42 are substantially similar to those of the first embodiment and will not again be described.

### <Specimen>

For example, in the diagnosis field, each of such items collected as test substances is handled as a specimen, as biological fluids such as the whole blood, blood plasma, blood serum, urine, feces, semen, and saliva, or a plant (or a portion thereof), an animal (or a portion thereof), or a melt of each thereof, and a solution prepared from a biological material such as a homogenate.

### <Sample ID Reading Part>

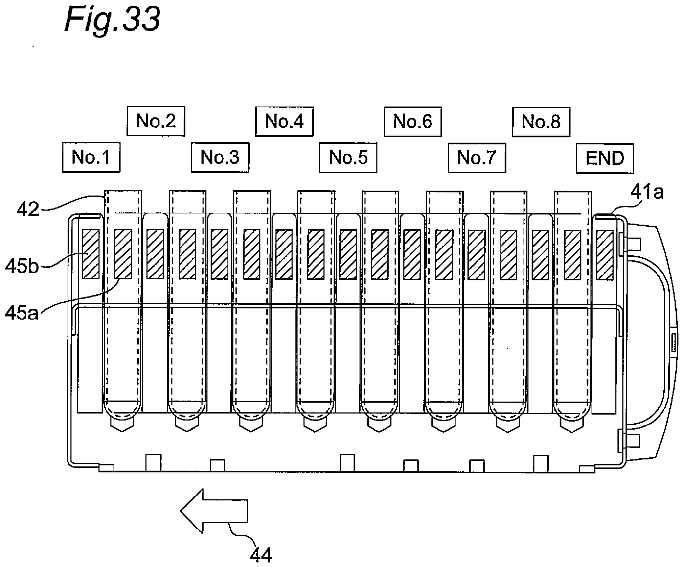

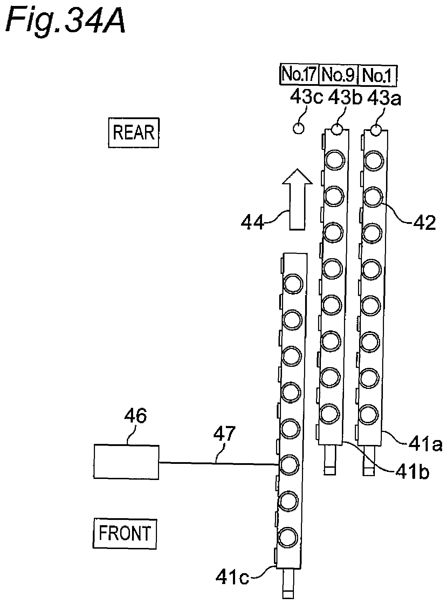

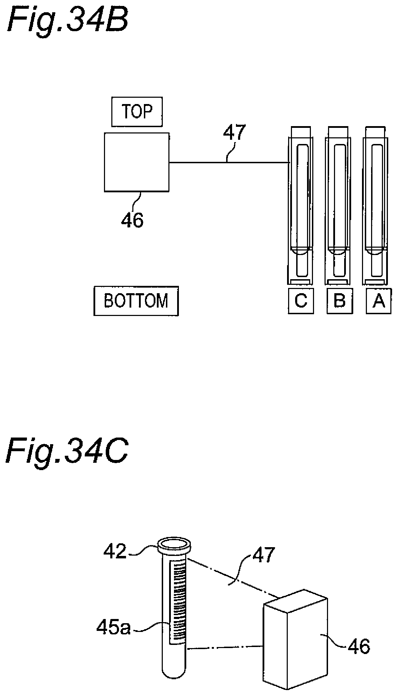

Fig. 33 is a side diagram of the sample rack 41a depicting a bar code 45a on a side face of each of the sample containers and a bar code 45b indicating the position of each of the sample rack 41a. Fig. 34A is a plan diagram of the state where the sample ID is read when the one sample rack 41c is accommodated in the sample setting part 12 in Fig. 4A. Fig. 34B is a plan diagram of Fig. 34A. Fig. 34C is a schematic diagram of the state where the bar code 45a on the side face of the sample container 42 is read by a bar code reader 46.

As depicted in Fig. 33, the bar code 45a is disposed that indicates the sample ID for each of the sample containers 42. The bar code 45b is disposed as a sample position ID at the position for each point to store the sample container 42 in the each point of the sample rack 41a. The sample position ID indicates the position for storing the sample container 42. In the example in Fig. 33, sequentially from the deepest position, No. 1, No. 2, No. 3, No. 4, No. 5, No. 6, No. 7, and No. 8 are present. This is because, when the sample rack 41a is accommodated in the lane along the setting direction 44, the bar code 45a of the sample container 42 at the tip of, that is, on the deep side of the sample rack 41a is read. The bar code 45b indicating the position has a bar code disposed thereon on the shallow side thereof that indicates "END" representing the end of the sample storage position.

As depicted in Fig. 34A, in the sample setting part 12, the code reader 46 is disposed to read the bar code indicating the sample ID of the sample container 42 from the direction that intersects the setting direction 44 of the sample racks 41a, 41b, and 41c. As depicted in Fig. 34A and Fig. 34B, the code reader 46 is disposed on the shallow side of the sample setting part 12. The code reader 46 corresponds to the sample ID reading part 18. The code reader 46 can read the bar codes of not only the sample rack 41c but also the sample racks 41a and 41b. The sample IDs and the sample position IDs can be read when the sample racks 41a, 41b, and 41c are set in the sample setting part 12.

As depicted in Fig. 34c, the sample ID may be indicated by a one-dimensional code such as, for example, a bar code or may be a two-dimensional code such as a QR code (a registered trademark). The code reader 46 can read the bar codes 45a and 45b by, for example, applying a red LED light beam 47 or the like and using an image sensor or the like.

The read sample ID and the read sample position ID are correlated with the corresponding collection tube ID by the ID managing part 17. Based on this correlation, the specimen accommodated in the sample container 42 is resolving-processed and the collection liquid including nucleic acid after the processing is accommodated in the corresponding collection tube 66.

### <Warming/Stirring Part>

The configuration of the warming/stirring part 13 of the nucleic acid separating apparatus 10 is similar to that in the plan diagram of Fig. 5A and the front diagram of Fig. 5B. The structure of each of the process tubes 53 arranged in the warming/stirring part 13 is similar to that in the perspective diagram of Fig. 5C. The state where the process tubes 53 are stirred in the warming/stirring part 13 is similar to that in the plan diagram of Fig. 6. The state of the warming/stirring part 13 warming the process tubes is similar to that in the front diagram of Fig. 7.

The warming/stirring part 13, the process tube 53, and the stirring and warming thereof are substantially similar to those of the first embodiment and will not again be described.

### <Nucleic Acid Extracting Part>

The configuration of the nucleic acid extracting part 14 of the nucleic acid separating apparatus 10 is similar to that in the plan diagram of Fig. 8A and the front diagram of Fig. 8B. The configuration of each of the filter cartridges 63 arranged in the nucleic acid extracting part 14 is similar to that in the schematic perspective diagram of Fig. 8C. The configuration of each of the collection tubes 66 arranged in the nucleic acid extracting part 14 is similar to that in the schematic perspective diagram of Fig. 8D.

The nucleic acid extracting part 14, the filter cartridge 63, and the collection tube 66 are substantially similar to those of the first embodiment and will not again be described.

### <Filter>

As depicted in Fig. 8C, the filter cartridge 63 includes the filter 64. The filter 64 (the nucleic acid adsorbing porous film) is substantially similar to that of the first embodiment and will not again be described.

### <Collection Tube>

As depicted in Fig. 8D, the collection tube 66 may include a lid that can close the upper face thereof. Nucleic acid is collected in this collection tube 66 together with the collection liquid.

### <Collection Tube ID Reading Part>

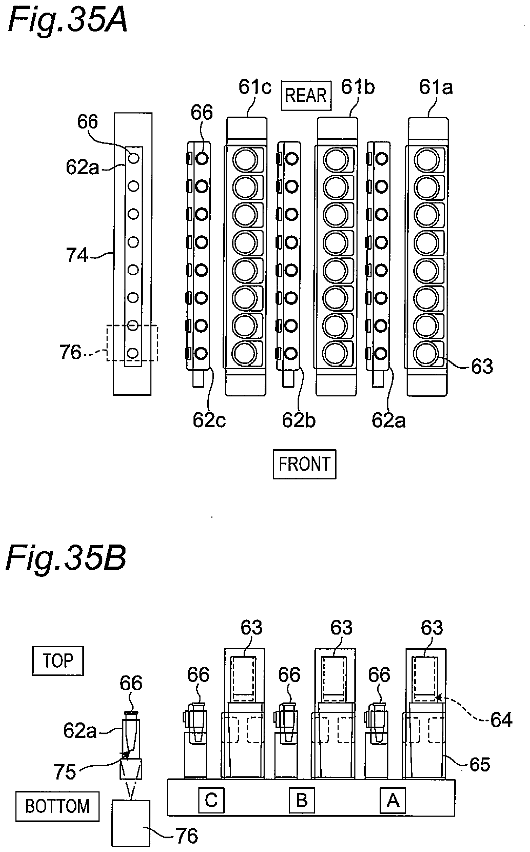

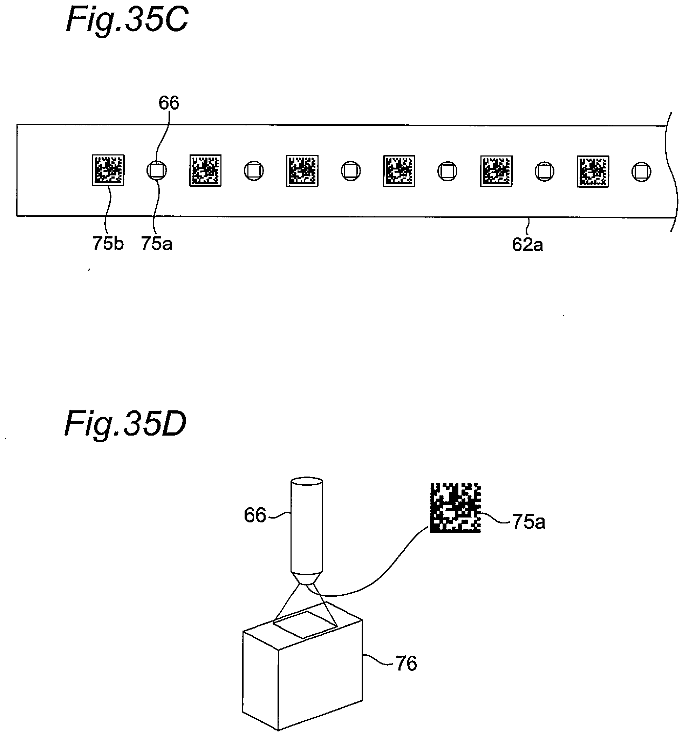

Fig. 35A is a plan diagram of an ID reading dedicated lane 74 for reading the collection tube ID of the collection tube 66 in the nucleic acid extracting part 14 in Fig. 8A. Fig. 35B is a front diagram of an installation point for a code reader 76 in the ID reading dedicated lane 74 in Fig. 35A. Fig. 35C is a bottom diagram of the collection tube rack 62a installed in the ID reading dedicated lane 74 in Fig. 35A. Fig. 35D is a schematic diagram of the state where a two-dimensional code 75a of the collection tube 66 is read by the code reader 76.

As depicted in Fig. 35A, the nucleic acid extracting part 14 has the ID reading dedicated lane 74 disposed therein to read the collection tube ID of the collection tube 66. Originally, before setting at the points for setting, the collection tube racks 62a, 62b, and 62c are sequentially set in the ID reading dedicated lane 74 and the collection tube IDs are read. As depicted in Fig. 35A and Fig. 35B, the code reader 76 is disposed in the lower portion on the shallow side of the ID reading dedicated lane 74. The code reader 76 corresponds to the collection tube ID reading part 19. The collection tube ID in the bottom portion of each of the collection tubes 66 is read when the collection tube racks 62a, 62b, and 62c are set in the ID reading dedicated lane 74.

As depicted in the bottom diagram of the collection tube rack 62a of Fig. 35C, the two-dimensional code 75a corresponding to the collection tube ID is disposed on the bottom face of the collection tube 66. A two-dimensional code 75b corresponding to the collection tube position ID that indicates the storage position of each of the collection tubes 66 may also be disposed on the bottom face itself of the collection tube rack 62a. In this case, the collection tube ID and the collection tube position ID can be read by reading the two-dimensional code 75a and the two-dimensional code 75b using the code reader 76.

As depicted in Fig. 35D, the collection tube ID may be indicated using, for example, the two-dimensional code 75a. The two-dimensional code can be disposed in a small area and can be disposed on the bottom face of the small collection tube 66. The code reader 76 can read the two-dimensional code 75 using, for example, an area camera.

### <Modification Example>

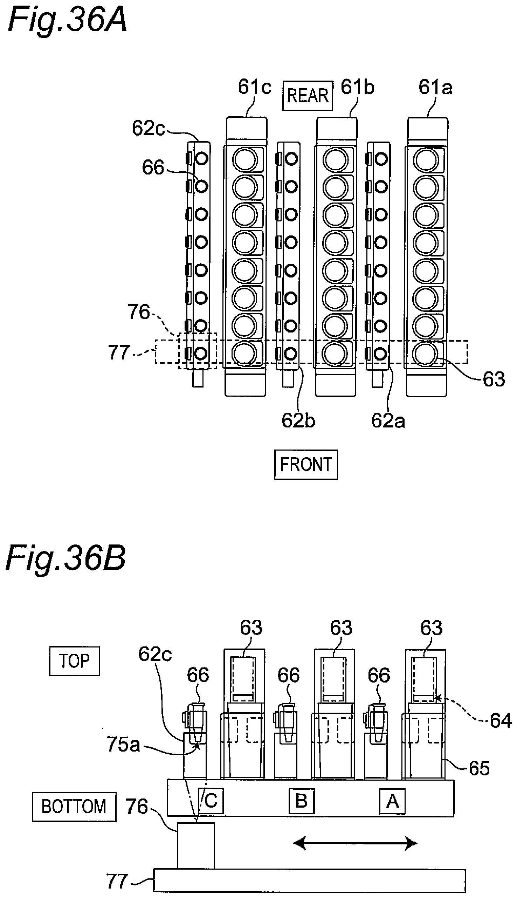

Fig. 36A is a plan diagram of a modification example that reads the collection tube ID without disposing the ID reading dedicated lane. Fig. 36B is a front diagram of an installation point for the code reader 76 in the modification example in Fig. 36A.

As depicted in Fig. 36A and Fig. 36B, different from the case in Fig. 35A, in this modification example, a code reader move lane 77 is disposed to intersect the setting direction in the lower portion on the shallow side of the nucleic acid extracting part 14 without disposing the ID reading dedicated lane. The one code reader 76 can thereby be moved along the code reader move lane 77 and the collection tube ID can be read from each of the collection tubes 66 for the collection tube racks 62a, 62b, and 62c. Similarly to the above, the two-dimensional code 75b corresponding to the collection tube position ID that indicates the position of the collection tube 66 may be disposed on the bottom face itself of each of the collection tube racks 62a, 62b, and 62c. The collection tube position ID can thereby be further read.

The read collection tube ID is correlated with the corresponding sample ID by the ID managing part 17. Based on this correlation, the collection liquid including nucleic acid originated from the specimen accommodated in the sample container 42 of the corresponding sample ID is accommodated in the corresponding collection tube 66.

When the collection tube position ID indicating the position of each of the collection tubes 66 is already read, the ID managing part 17 can correlate the collection tube ID and the corresponding collection tube position ID with each other. The position of each of the collection tubes 66 can thereby be identified in the collection tube racks 62a, 62b, and 62c.

### <About Move of Filter Cartridge>

The state where the filter cartridge 63 is moved to the position above the collection tube 66 for collection of nucleic acid, to separate nucleic acid in the nucleic acid extracting part 14 is similar to that in Fig. 9A and Fig. 9B.

The filter cartridges 63 need to be moved to the position above the collection tubes 66 before the collection of nucleic acid. The move of the filter cartridges 63 is substantially similar to that of the first embodiment and will not again be described.

### <Dispensing and Pressurizing Unit>

The configuration of the dispensing and pressurizing unit 15 of the nucleic acid separating apparatus 10 is similar to that in the schematic perspective diagram of Fig. 10A. The liquid supply nozzles 21a and 21b of the dispensing and pressurizing unit 15 are similar to those in the partial diagram of Fig. 10B. The pressurizing heads 23a and 23b of the dispensing and pressuring unit 15 are similar to those in the partial diagram of Fig. 10C. The suction/discharge nozzles 22a and 22b of the dispensing and pressurizing unit 15 are similar to those in the partial diagram of Fig. 10D. Fig. 11A is the plan diagram of the dispensing and pressurizing unit 15. Fig. 11B is the side diagram of the dispensing and pressurizing unit 15. Fig. 11C is the front diagram of the dispensing and pressurizing unit 15.

The dispensing and pressurizing unit 15 and the receiving tray are substantially similar to those of the first embodiment and will no again be described.

### <Suction and Discharge Mechanism>

The suction and discharge mechanism (the pipette) 22a and 22b is characterized in that the mechanism does not dispense the cleaning liquid and the like each through a pipe directly connected to a bottle including the cleaning liquid or the like but injects the liquid through the dispensing chip 32 attached to the tip thereof. The suction and discharge mechanism (the pipette) 22a and 22b is substantially similar to that of the first embodiment and will not again be described.

### <Liquid Supply Mechanism>

Fig. 12A is the plan diagram of the liquid supply nozzles 21a and 21b that each discharge a liquid in the dispensing and pressurizing unit 15, and the tube pumps 71 and the reagent bottles 36f connected to the liquid supply nozzles (the liquid supply mechanism) 21a and 21b. Fig. 12B is the side diagram of the dispensing and pressurizing unit 15. Fig. 12C is the front diagram of the dispensing and pressurizing unit 15. The liquid supply nozzles 21a and 21b supply a reagent (such as, for example, the cleaning liquid) from the reagent bottles 36f by the tube pumps 71. The supply amount by each of the liquid supply nozzles 21a and 21b in one session is, for example, 12 ml or smaller and the liquid may be supplied in units each of 0.25 ml.

### <Pressurizing Head (Pressurized Gas Supply Mechanism)>

Fig. 13A is the plan diagram of the pressurizing heads 23a and 23b in the dispensing and pressurizing unit 15, and pipes connected to the pressurizing heads 23a and 23b. Fig. 13B is the side diagram of the dispensing and pressurizing unit 15. Fig. 13C is the front diagram of the dispensing and pressurizing unit 15.

The pressurizing heads 23a and 23b are respectively connected to the air filters 81a and 81b that are disposed at the ventilating opening through the pulse motors 82a and 82b, the pressurizing pumps 83a and 83b, and the pressure sensors 86a and 86b. The set range of the pressure is, for example, 30 kPa to 160 kPa. For example, air or nitrogen is usable as the pressurized gas.

The pressurizing heads 23a and 23b are arranged to avoid their interference on the operations of the suction and discharge mechanism 22a and 22b, and the liquid supply nozzles 21a and 21b.

### <Move Mechanism>

The move mechanism 24 can move the overall dispensing and pressurizing unit 15 to the position above the containers 53 and 63 in the vertical direction. For example, as depicted in Fig. 1A and Fig. 1B, the move mechanism 24 can use the X-axis actuator 24a, the Y-axis actuator 24b, and the Z-axis actuator 24c.

### <Control Unit>

The control unit 16 controls the dispensing chip/reagent setting part 11, the sample setting part 12, the warming/stirring part 13, the nucleic acid extracting part 14, and the dispensing and pressurizing unit 15 that constitute the nucleic acid separating apparatus 10. The control part 16 may be, for example, a computer. The computer only has to include the physical components such as, for example, the CPU 1, the memory 2, the storing part 3, the displaying part 4, the input part 5, and the interface 6. The ID managing part 17 may be realized by hardware and software that are commonly used with the control part 16.

### <Nucleic Acid Separation Method>

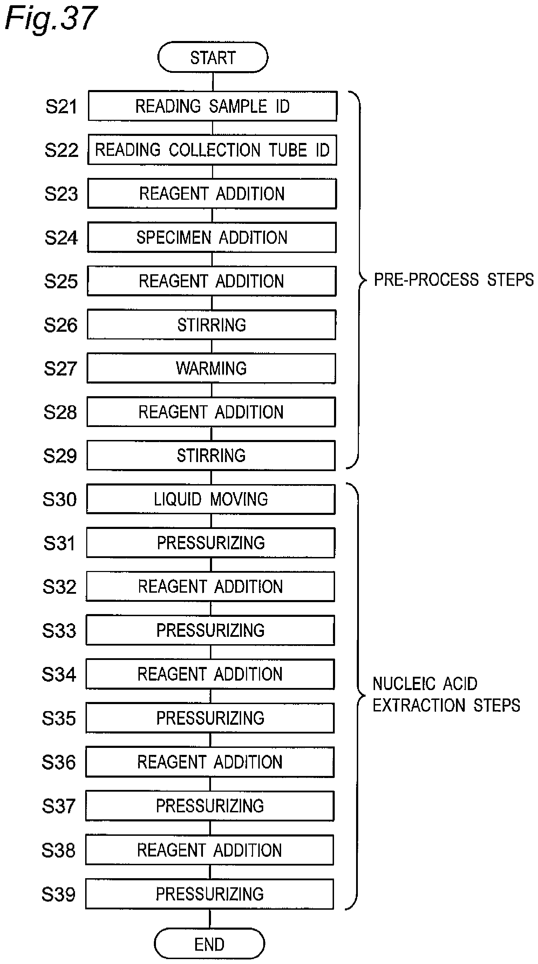

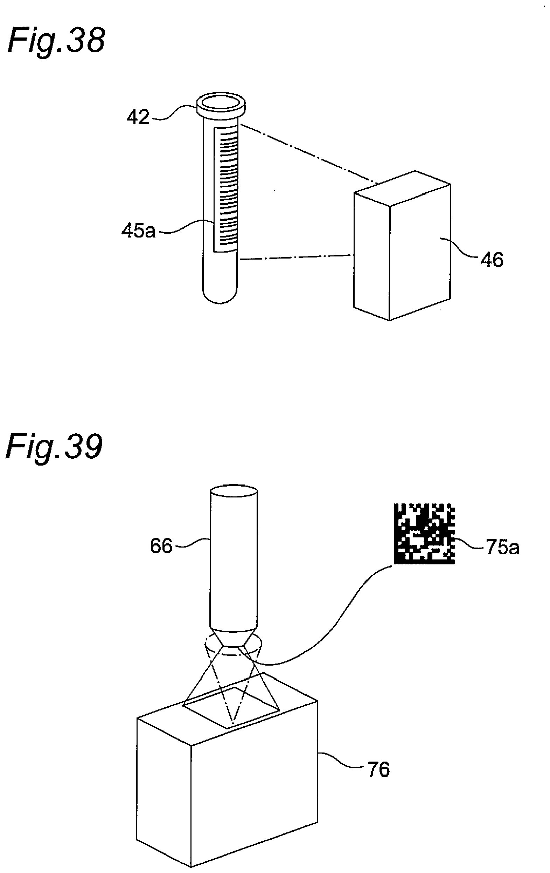

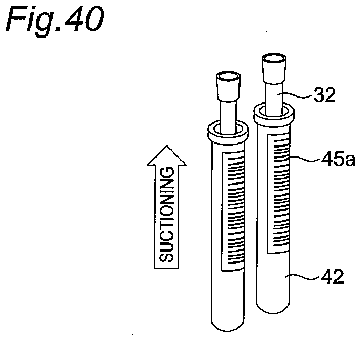

Fig. 37 is a flowchart of steps of a nucleic acid separation method according to the second embodiment. In Fig. 37, steps from a sample ID reading step (S21) to a stirring step (S29) are the pre-process (the resolving process) steps and steps from a liquid moving step (S30) to a pressurizing step (S39) are the nucleic acid extraction (the separation and collection) steps.
(1) The sample ID of the sample container 42 is read from the direction intersecting the setting direction 44 when the sample racks 41a, 41b, and 41c are set in the sample setting part 12 (Fig. 38) (the sample ID reading step S21). The sample ID may be indicated by, for example, the bar code 45a. As depicted in Fig. 38, the bar code 45a may be disposed along the longitudinal direction of the sample container 42.
(2) The collection tube ID of the collection tube 66 is read when the collection tube racks 62a, 62b, and 62c are set in the nucleic acid extracting part 14 (Fig. 39) (a collection tube ID reading step S22). The collection tube ID may be indicated by, for example, the two-dimensional code 75a. The two-dimensional code can be disposed in a small area and can be disposed on the bottom face of the small collection tube 66. The ID managing part 17 correlates the sample ID and the corresponding collection tube ID with each other. Based on this correlation, the specimen liquid including nucleic acid accommodated in the sample container 42 is thereafter resolving-processed (S27) and the collection liquid including nucleic acid after the processing is accommodated in the corresponding collection tube 66 (S39).
(3) The reagent (the pre-process enzyme) is suctioned from the reagent container 36 to the dispensing chip 32 (Fig. 15A) and is dispensed into the process tube 53 (Fig. 15B) (a reagent addition step S23).
(4) The specimen (the whole blood) is suctioned from the sample container (a blood collection tube, 42) to the dispensing chip 32 (Fig. 40) and is dispensed into the process tube 53 (Fig. 16B) (a specimen addition step S24).
(5) The reagent (the solution liquid) is suctioned from the reagent container 36 to the dispensing chip 32 (Fig. 17A) and is dispensed into the process tube 53 (Fig. 17B) (a reagent addition step S25).
(6) The process tube 53 is stirred (Fig. 18) (a stirring step S26). The stirring of the process tube 53 is executed at the rotation number of the motor of 1,500 rpm or smaller. The amplitude thereof is 1 mm to 10 mm.
(7) The process tube 53 is warmed (Fig. 19) (a warming step S27). The warming is executed in, for example, a temperature range from 20°C to 60°C.
   The "resolving process" is processing executed using water solution including a reagent (such as, for example, a solution including chaotropic salt or guanidine salt, a surfactant, and a protein-degrading enzyme) that melts the cell membrane and the nucleic membrane and thereby causes the nucleic acid to be soluble. This "resolving process" is substantially similar to that of the first embodiment and will no again be described.
(8) The reagent (ethanol) is suctioned from the reagent container 36 to the dispensing chip 32 (Fig. 20A) and is dispensed into the process tube 53 (Fig. 20B) (a reagent addition step S28).
(9) The process tube 53 is stirred (Fig. 21) (the stirring step S29). The stirring of the process tube 53 is executed at the rotation number of the motor of 1,500 rpm or smaller. The amplitude thereof is 1 mm to 10 mm.
(10) The specimen liquid including resolving-processed nucleic acid is suctioned from the process tube 53 to the dispensing chip 32 (Fig. 22A) and is dispensed into the filter cartridge 63 (Fig. 22B) (the liquid move step S30). The specimen liquid including resolving-processed nucleic acid can thereby be injected into the filter cartridge 63.
(11) The filter cartridge 63 is filtered under pressure (Fig. 23) (a pressurizing step S31). The filtering under pressure is executed by introducing a pressurized air into the filter cartridge 63 to pressurize its inside. In this case, the inside of the filter cartridge 63 is maintained to be in a sealed state. The specimen liquid is thereby caused to pass through the filter 64 and nucleic acid is adsorbed by the filter 64. The liquid-state portion passing through the filter 64 is discharged through the waste tube 65 arranged under the filter cartridge 63. When the overall specimen liquid passes through the filter 64, the pressure is lowered to a liquid discharge completion pressure or lower, and the end of the extraction is detected by the pressure sensors 86a and 86b at all the filter cartridges 63. For example, the pressurizing heads 23a and 23b are thereafter moved to be lifted, the sealed state is cancelled, and the pressurizing step comes to an end.
(12) The reagent (the cleaning liquid) is dispensed from the liquid supply nozzles 21a and 21b to the filter cartridge 63 (Fig. 24) (a reagent addition step S32).

### <Cleaning Liquid>

The "cleaning liquid" has a function of washing away any impurity in the specimen liquid adhering to the filter 64 together with nucleic acid, and has a composition for any impurity to depart leaving the adsorption of nucleic acid as it is. The "cleaning liquid" is similar to that of the first embodiment and will not again be described.
(13) The filter cartridge 63 is filtered under pressure (Fig. 25) (a pressurizing step S33). The filtering under pressure is executed by introducing a pressurized air into the filter cartridge 63 to pressurize its inside. The impurities other than nucleic acid are cleaned to be removed causing the filter 64 to retain nucleic acid as it is. The liquid-state portion passing through the filter 64 is discharged through the waste tube 65 connected to the filter cartridge 63.
(14) The reagent (the cleaning liquid) is dispensed from the liquid supply nozzles 21a and 21b to the filter cartridge 63 (Fig. 26) (a reagent addition step S34).
(15) The filter cartridge 63 is filtered under pressure (Fig. 27) (a pressurizing step S35).
(16) The reagent (the cleaning liquid) is dispensed from the liquid supply nozzles 21a and 21b to the filter cartridge 63 (Fig. 28) (a reagent addition step S36).
(17) The filter cartridge 63 is filtered under pressure (Fig. 29) (a pressurizing step S37). The combination of the reagent addition step S32 and the pressurizing step S33, that of the reagent addition step S34 and the pressurizing step S35, and that of the reagent addition step S36 and the pressurizing step S37 are repeated for three times in the above. The repetition is not limited to the above example and at least one combination of the reagent addition step and the pressurizing step only has to be executed.
(18) The filter cartridges 63 are moved from the filter cartridge racks 61a, 61b, and 61c to the collection tube racks 62a, 62b, and 62c (Fig. 9A and Fig. 9B). For example, as indicated by the arrow 67 in Fig. 9B, the filter cartridge 63 is moved upward. As indicated by the arrow 68 therein, the filter cartridge 63 is next horizontally moved to the position above the collection tube 66. As indicated by the arrow 69 therein, the filter cartridge 63 is next moved downward, and the filter cartridge 63 is arranged on the collection tube 66. The filter cartridges 63 are similarly moved sequentially and the filter cartridges 63 are each arranged on one of the collection tubes 66.
(19) The reagent (the collection liquid) is suctioned from the reagent container 36 to the dispensing chip 32 (Fig. 30A) and is dispensed to the filter cartridge 63 (Fig. 30B) (a reagent addition step S38).

### <Collection Liquid>

The "collection liquid" advantageously has a low salt concentration. Especially, a solution having a salt concentration of 0.5 M or lower such as, for example, purified distilled water or a TE buffer is used. "pH" of the collection liquid is preferably pH2 to pH11 and further preferably pH5 to pH9.

(20) The filter cartridge 63 is filtered under pressure (Fig. 31) (the pressurizing step S39). The filtering under pressure is executed by introducing a pressurized air into the filter cartridge 63 to pressurize its inside. The bonding force between the filter 64 and nucleic acid can be weakened by the collection liquid to cause nucleic acid adsorbed by the filter 64 to leave therefrom, and nucleic acid can be collected into the collection tube 66 together with the collection liquid. The collection tube 66 may thereafter be closed by a lid when necessary.

The collection liquid including nucleic acid can be collected by the above operation. For nucleic acid in the collection tube 66, a nucleic acid analysis process and the like are next executed.

According to the nucleic acid separating apparatus 10 and the nucleic acid separation method, for the plural sample containers 42, steps from the pre-process steps to the nucleic acid extraction step are sequentially and continuously executed for each of the sample containers. The manually executed traditional pre-process steps therefore do not need to be executed.

In this second embodiment, plural sets of the sample container 42, the process tube 53, and the filter cartridge 63 are included while the number of sets is not limited to these. For example, the nucleic acid separating apparatus 10 according to the second embodiment is applicable to one set of the sample container 42, the process tube 53, and the filter cartridge 63.

The nucleic acid separating apparatus 10 may include a UV applying part (not depicted) that disinfects by applying a UV light beam to the inside of the apparatus. The UV applying part may be configured to apply a UV light beam moving inside the apparatus in, for example, the x- and the y-directions. The inside of the apparatus can thereby be maintained to be clean.

This disclosure includes a proper combination of any optional embodiment(s) and/or Example(s) of the above various embodiments and/or Examples, and the effects to be achieved by the embodiment(s) and/or Examples can be achieved.

According to the nucleic acid separating apparatus of the present invention, a dispensing mechanism that dispenses a specimen, a reagent, or a specimen liquid after a resolving process through a dispensing chip, and a pressurized gas supply mechanism that supplies a pressurized gas are included therein. Plural steps such as the dispensing and the pressurizing can thereby be automated, and not only a nucleic acid extraction step but also the traditionally manually executed pre-process steps can be automated.

According to the nucleic acid separating apparatus of another example of the present invention, an ID managing part can correlate a sample ID of a sample container, and a collection tube ID of the corresponding collection tube with each other. Any mistaken recognition for the sample container and the corresponding collection tube can thereby be suppressed.

### REFERENCE NUMERALS LIST

- 10: nucleic acid separating apparatus
- 11: dispensing chip/reagent setting part
- 12: sample setting part
- 13: warming/stirring part
- 14: nucleic acid extracting part
- 15: dispensing and pressurizing unit
- 16: control part
- 17: ID managing part
- 18: sample ID reading part
- 19: collection tube ID reading part
- 21, 21a, 21b: reagent addition nozzle (liquid supply mechanism)
- 22, 22a, 22b: suction/discharge nozzle (suction and discharge mechanism)
- 23, 23a, 23b: pressurizing head (pressurized gas supply mechanism)
- 24, 24a, 24b, 24c: move mechanism
- 25: receiving tray
- 26a, 26b: first dispensing chip attaching part
- 27a, 27b: second dispensing chip attaching part
- 28: work sensing sensor
- 31a, 31b, 31c: dispensing chip rack
- 32, 32a, 32b, 32c, 32d: dispensing chip
- 33: dispensing chip ejector part
- 34: dispensing chip discarding box
- 35: reagent container tray
- 36, 36a, 36b, 36c, 36d, 36e, 36f: reagent container
- 41a, 41b, 41c: sample rack
- 42, 42a, 42b, 42c: sample container (blood collection tube)
- 43a, 43b, 43c: sample rack presence or absence sensing sensor
- 44: sample rack setting direction
- 45a: bar code (sample ID)
- 45b: bar code (sample position ID)
- 46: code reader
- 47: red LED light beam
- 51: stirring mechanism (stirring unit)
- 52: process tube rack
- 53: process tube (lysate tube)
- 54: neck portion
- 55: bottom portion
- 56: rotation direction
- 58: heater
- 61a, 61b, 61c: filter cartridge rack
- 62a, 62b, 62c: collection tube rack
- 63: filter cartridge
- 64: filter
- 65: waste tube
- 66: collection tube
- 67: move to be lifted
- 68: move in the horizontal direction
- 69: downward move
- 71: tube pump
- 72: pipe
- 74: ID reading dedicated lane
- 75: two-dimensional code
- 76: code reader
- 77: code reader move lane
- 81a, 81b: air filter
- 82a, 82b: pulse motor
- 83a, 83b: pressurizing pump
- 86a, 86b: pressure sensor

## Claims

1. A nucleic acid separating apparatus comprising:
a stirring mechanism that stirs and resolving-processes a specimen and a reagent injected into a process tube;
a pressurized gas supply mechanism that supplies a pressurized gas to a filter cartridge having a specimen liquid including nucleic acid, the specimen liquid being injected in the filter cartridge after the resolving-process, to separate and collect nucleic acid from the specimen liquid;
a dispensing mechanism that injects the specimen, a reagent, or the specimen liquid after the resolving-process into the process tube and the filter cartridge; and
a move mechanism that moves the pressurized gas supply mechanism and the dispensing mechanism,
wherein the dispensing mechanism comprises:
a suction and discharge mechanism that is capable of attaching a dispensing chip to the suction and discharge mechanism and detaching the dispensing chip from the suction and discharge mechanism, the suction and discharge mechanism being capable of suctioning and discharging a liquid through the dispensing chip; and
a liquid supply mechanism that discharges a liquid from a nozzle.

2. The nucleic acid separating apparatus according to claim 1, wherein the suction and discharge mechanism suctions or discharges a gas in the dispensing chip, and suctions or discharges a liquid into the dispensing chip, and
wherein the suction and discharge mechanism comprises:
a first dispensing chip attaching part that includes an O-ring having a first caliber at a tip of the first dispensing chip attaching part, the first dispensing chip attaching part being capable of attaching a first dispensing chip having the first caliber to the first dispensing chip attaching part and detaching the first dispensing chip from the first dispensing chip attaching part; and
a second dispensing chip attaching part that includes an O-ring having a second caliber larger than the first caliber to be concentric with the O-ring having the first caliber, the second dispensing chip attaching part being capable of attaching a second dispensing chip having the second caliber to the second dispensing chip attaching part and detaching the second dispensing chip from the second dispensing chip attaching part.
